(19) Europäisches Patentamt
European Patent Office
Office européen des brevets

(11) EP 3 483 240 A1

(12) **EUROPEAN PATENT APPLICATION**

(43) Date of publication:
**15.05.2019 Bulletin 2019/20**

(21) Application number: **17201323.7**

(22) Date of filing: **13.11.2017**

(51) Int Cl.:
***C11D 1/94*** (2006.01)
***C07K 14/46*** (2006.01)
***C11D 11/00*** (2006.01)
***C07K 14/77*** (2006.01)
***C11D 3/38*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **The Procter & Gamble Company**
**Cincinnati, OH 45202 (US)**

(72) Inventors:
- **BETTIOL, Jean-Luc Philippe**
  **1853 Strombeek-Bever (BE)**
- **GONZALES, Denis Alfred**
  **1853 Strombeek-Bever (BE)**
- **VELASQUEZ, Juan Esteban**
  **Cincinnati, Ohio 45202 (US)**
- **GEARY, Nicholas William**
  **Cincinnati, Ohio 45202 (US)**

(74) Representative: **Kellenberger, Jakob**
**NV Procter & Gamble**
**Services Company S.A.**
**Temselaan 100**
**1853 Strombeek-Bever (BE)**

(54) **DETERGENT COMPOSITION COMPRISING MODIFIED PROTEINS**

(57) The present invention is directed to a detergent composition comprising a modified protein selected from the group consisting of modified ovalbumin, modified ovomucin, modified ovoglobulin, and mixtures thereof, and a surfactant system. Methods of making and using such compositions are also provided.

EP 3 483 240 A1

**Description**

REFERENCE TO A SEQUENCE LISTING

This application contains Sequence Listings in computer readable form. The computer readable form is incorporated herein by reference.

FIELD OF THE INVENTION

The present invention relates to a detergent composition comprising a modified protein selected from the group consisting of modified ovalbumin, modified ovomucin, modified ovoglobulin, and mixtures thereof, and a surfactant system. The composition provides one or more benefits, including good cleaning particularly good grease emulsification, long lasting suds especially in presence of greasy soils and surface modification that can contribute to second time cleaning benefits, improved drying, improved shine.

BACKGROUND OF THE INVENTION

Detergent compositions should provide good soil and/or grease cleaning while presenting a good suds profile in particular a long lasting suds profile especially in the presence of greasy soils. Users usually see suds as an indicator of the performance of the detergent composition. Moreover, the user of a detergent composition may also use the suds profile and the appearance of the suds (e.g., density, whiteness) as an indicator that the wash solution still contains active cleaning ingredients. This is particularly the case for manual washing, also referred to herein as hand-washing, where the user usually doses the detergent composition depending on the suds remaining and renews the wash solution when the suds subsides or when the suds does not look thick enough. Thus, a detergent composition, particularly a manual wash detergent composition that generates little or low density suds would tend to be replaced by the user more frequently than is necessary. Thus, it is desirable for a detergent composition to provide "good sudsing profile", which includes good suds height and/or density as well as good suds duration during the initial mixing of the composition with water and/or during the entire washing operation.

Albumins are a family of globular proteins, hence consisting of long folded chains of amino acids. The family includes albumins present in egg white. In particular, chicken egg albumin is a phosphorylated-glycoprotein. According to U.S. Pat. No. 4,434,089, unmodified *(i.e.,* undegraded) albumin proteins, in particular undegraded casein, albumen and gelatin, present in detergent compositions comprising dialkyl sulphosuccinate surfactants would increase suds performance, especially under hard water conditions. U.S. Pat. No. '089 also states that the addition of these unmodified *(i.e.,* undegraded) albumin proteins to dishwashing detergents based on alkylbenzene sulphates would not to lead to enhancement of suds performance. Furthermore, the addition of partially modified *(i.e.,* degraded) albumin proteins to detergent compositions does not result in enhancement of suds performance neither, as disclosed in GB Pat. No. 1 160 485. However, the inclusion of "modified" albumin proteins has not been disclosed, particularly in the context of detergent compositions for improving sudsing profile. While unmodified proteins might positively impact the suds duration of a detergent composition even in presence of greasy soils, the Applicants believe that through modifying the proteins a more optimized affinity of the protein to emulsify greasy soils can be achieved, as such releasing the surfactants more from the oil-water interface and rendering them available to continue stabilizing the suds at the air water interface.

Accordingly, the need remains for an improved detergent composition comprising a modified albumin protein which has a further improved sudsing profile, particularly at low modified albumin protein concentrations in the detergent composition. The need also exists for an improved detergent composition, when used in a manual-washing process, the composition should also provide a pleasant washing experience, *i.e.,* good feel on the user's hands during the wash. The composition should also be easy to rinse. Further it is desirous that the improved detergent composition is stable and will not phase separate, resulting in greater shelf-life of the product. It is also desirable that the detergent composition provides surface modification, contributing to shine and/or improved second time cleaning. There is also the desire to reduce the amount of surfactants without negatively impacting sudsing nor grease cleaning and emulsification profile. Thus, there is the need to find new compositions that improve cleaning, suds longevity and improved after cleaning benefits in hand washing conditions. The Applicant discovered that some or all of the above-mentioned needs can be at least partially fulfilled through the improved detergent composition as described herein below.

SUMMARY OF THE INVENTION

The present invention meets one or more of these needs based on the surprising discovery that by formulating a detergent composition comprising a modified protein and a surfactant system, such a composition exhibits good sudsing profile, particularly desirable suds volume and/or sustained suds stabilization, especially in the presence of

greasy soils. It also provides good grease cleaning and emulsification benefits and can also provide surface modifications facilitating next time cleaning benefit.

According to a first aspect, the present invention is directed to a detergent composition comprising a modified protein preferably selected from the group consisting of modified ovalbumin, modified ovomucin, modified ovoglobulin, and mixtures thereof, and a surfactant system. Preferably the modified ovalbumin is derived from an unmodified ovalbumin which has at least at least 50%, preferably at least 60%, preferably at least 70%, preferably at least 80%, preferably at least 85%, preferably at least 90%, preferably at least 95%, preferably at least 98% or even 100% amino acid identity to native ovalbumin protein (SEQ ID NO: 1). Preferably modified ovomucin is derived from an unmodified ovomucin which has at least 50%, preferably at least 60%, preferably at least 70%, preferably at least 80%, preferably at least 85%, preferably at least 90%, preferably at least 95%, preferably at least 98% or even 100% amino acid identity to at least one native ovomucin protein selected from the group consisting of SEQ ID NO: 2, SEQ ID NO: 4, and SEQ ID NO: 5, preferably SEQ ID NO: 2 or SEQ ID NO: 5. Preferably the modified ovoglobulin is derived from an unmodified ovoglobulin which has at least 50%, preferably at least 60%, preferably at least 70%, preferably at least 80%, preferably at least 85%, preferably at least 90%, preferably at least 95%, preferably at least 98% or even 100% amino acid identity to a native ovoglobulin protein (SEQ ID NO: 3).

Preferably the detergent composition is a manual-washing composition. Preferably the detergent composition is for manual dishwashing. Preferred compositions are in the form of a liquid.

In another aspect, the present invention is directed to a method of manually washing dishware comprising the steps of delivering a detergent composition of the invention into a volume of water to form a wash solution and immersing the dishware in the solution.

In yet another aspect, there is provided a method of manual washing comprising the step of: delivering the detergent composition of the invention to a volume of water and immersing soiled articles in the water. When the composition of the invention is used according to this method a good sudsing profile, with a long lasting effect is achieved.

In yet another aspect, the present invention is directed to a method of manually washing dishware comprising the steps of: i) delivering a composition of the present invention onto the dishware or a cleaning implement; ii) cleaning the dishware with the composition in the presence of water; and iii) optionally, rinsing the dishware. Preferably, the composition of the present invention is used in neat form *(i.e.,* direct application) since greater benefits in terms of grease cleaning are obtained when the composition is directly applied on the soiled surface or on a cleaning implement, such as a sponge, to be used to clean the soiled surface.

There is also provided the use of a modified protein selected from the group consisting of modified ovalbumin, modified ovomucin, modified ovoglobulin, and mixtures thereof to provide increased suds longevity or increased grease emulsification in an aqueous wash liquor during a washing process.

Preferably the manual washing is dishwashing and the soiled articles comprise soiled dishware. As used herein, "dishware" includes cookware and tableware.

The elements of the composition of the invention described in relation to the first aspect of the invention apply *mutatis mutandis* to the other aspects of the invention.

These and other features, aspects and advantages of the present invention will become evident to those skilled in the art from the detailed description which follows.

## DETAILED DESCRIPTION OF THE INVENTION

### Definitions

As used herein, the articles "a" and "an" when used in a claim, are understood to mean one or more of what is claimed or described.

As used herein, the term "amino acid identity" means the identity between two or more amino acid sequences and is expressed in terms of the identity or similarity between the sequences. Sequence identity can be measured in terms of percentage identity; the higher the percentage, the more identical the sequences are. The percentage identity is calculated over the length of comparison. For example, the amino acid identity is typically calculated over the entire length of a sequence aligned against the entire length of the reference sequence (e.g., SEQ ID NO: 1, SEQ ID NO: 2, SEQ ID NO: 3, SEQ ID NO: 4, SEQ ID NO: 5). Methods of alignment of sequences for comparison are well known in the art and identity can be calculated by many known methods. Various programs and alignment algorithms are described in the art. It should be noted that the terms ’sequence identity’ and ’sequence similarity’ can be used interchangeably.

As used herein, the term "detergent composition" refers to a composition or formulation designed for cleaning soiled surfaces. Such compositions include but are not limited to, dishwashing compositions, laundry detergent compositions, fabric softening compositions, fabric enhancing compositions, fabric freshening compositions, laundry pre-wash, laundry pretreat, laundry additives, spray products, dry cleaning agent or composition, laundry rinse additive, wash additive, post-rinse fabric treatment, ironing aid, hard surface cleaning compositions, unit dose formulation, delayed

delivery formulation, detergent contained on or in a porous substrate or nonwoven sheet, and other suitable forms that may be apparent to one skilled in the art in view of the teachings herein. Such compositions may be used as a pre-cleaning treatment, a post-cleaning treatment, or may be added during the rinse or wash cycle of the cleaning process. The detergent compositions may have a form selected from liquid, powder, single-phase or multi-phase unit dose or pouch form, tablet, gel, paste, bar, or flake. Preferably the composition is for manual-washing. Preferably, the detergent composition of the present invention is a dishwashing detergent. Preferably the composition is in the form of a liquid.

As used herein the term "fragment" means an amino acid sequence of at least 30, 60, 100, 150 contiguous amino acids of the reference sequences or any integer there between.

As used herein the term "increased suds longevity" means an increase in the duration of visible suds in a washing process cleaning soiled articles using the composition comprising a modified protein, compared with the suds longevity provided by the same composition and process in the absence of the modified protein or relative to the un-modified albumin protein.

As used herein, the term "modified protein" refers to a protein that has been chemically or physically modified. The term "chemically modified" protein in the context of the present invention is understood to mean proteins that are obtained by chemical reaction of the reactive groups of proteins. In particular the carboxyl, hydroxy, amine, imidazole, and/or thiol groups of the side chains of the amino acids of the protein. For example, chemically modified protein may include proteins that are partially or completely denatured, partially or completely desugarized, partially or completely esterified, partially hydrolyzed, or combinations thereof. The term "physically modified" protein in the context of the present invention is understood to mean proteins that are modified by physical effect, such as for example, by heat, light, fractionation, sonication, etc.

As used herein, the term "native" refers to a protein that is the wild-type sequence, and has been neither chemically or physically modified.

As used herein, the term "next time cleaning benefit" means the surface to be cleaned could be treated with a composition which would assist in easier removal of soil and/or stains during subsequent cleaning.

As used herein, the term "soiled surfaces" refers non-specifically to any type of flexible material consisting of a network of natural or artificial fibers, including natural, artificial, and synthetic fibers, such as, but not limited to, cotton, linen, wool, polyester, nylon, silk, acrylic, and the like, as well as various blends and combinations. Soiled surfaces may further refer to any type of hard surface, including natural, artificial, or synthetic surfaces, such as, but not limited to, tile, granite, grout, glass, composite, vinyl, hardwood, metal, cooking surfaces, plastic, and the like, as well as blends and combinations, as well as dishware. Key targeted soiled surfaces by this application are soiled dishware.

As used herein, the "unmodified protein" refers to a protein that has neither been treated by chemical nor physical methods.

As used herein, the term "variant" of the modified proteins means an amino acid sequence when the modified protein is modified by, or at, one or more amino acids (for example 1, 2, 3, 4, 5, 6, 7, 8, 9 or 10 or more amino acid modifications) selected from substitutions, insertions, deletions and combinations thereof. The variant may have "conservative" substitutions, wherein a substituted amino acid has similar structural or chemical properties to the amino acid that replaces it, for example, replacement of leucine with isoleucine. A variant may have "non-conservative" changes, for example, replacement of a glycine with a tryptophan. Variants may also include sequences with amino acid deletions or insertions, or both. Guidance in determining which amino acid residues may be substituted, inserted, or deleted without abolishing the activity of the protein may be found using computer programs well known in the art. Variants may also include truncated forms derived from a wild-type protein, such as for example, a protein with a truncated N-terminus.

As used herein, the term "water hardness" or "hardness" means uncomplexed cations ion *(i.e.,* $Ca^{2+}$ or $Mg^{2+}$) present in water that have the potential to precipitate under alkaline conditions, and thereby diminishing the surfactancy and cleaning capacity of surfactants. Further, the terms "high water hardness" and "elevated water hardness" can be used interchangeably and are relative terms for the purposes of the present invention, and are intended to include, but not limited to, a hardness level containing at least 12 grams of calcium ion per gallon water (gpg, "American grain hardness" units).

## Detergent Composition

A preferred detergent composition is a manual dishwashing composition, preferably in liquid form. Preferably the detergent composition comprises from 30% to 95%, preferably from 40% to 90%, more preferably from 50% to 85%, by weight of the composition of a liquid carrier in which the other essential and optional components are dissolved, dispersed or suspended. One preferred component of the liquid carrier is water.

Preferably the pH of the detergent composition, measured as a 10% product concentration in demineralized water at 20 °C, is adjusted to between 3 and 14, more preferably between 4 and 13, more preferably between 6 and 12 and most preferably between 8 and 10. The pH of the detergent composition can be adjusted using pH modifying ingredients known in the art.

Modified Proteins

Ovalbumin is, a globular phosphoglycoprotein with a molecular weight of 45 KDa and an isoelectric point (pI) of 4.5. Ovalbumin contains 385 amino-acid residues of which about half are hydrophobic. It is believed that ovalbumin absorbs at the water air and/or the oil water interface to form an interfacial network that acts as a suds stabilizer and/or a grease emulsifier.

Ovomucin is a large sulfated glycoprotein with a molecular weight varying from 5.5 to 8.3 MDa and is having a isolectrical point of 4.5-5. It is believed to emulsify grease and improve suds stability and participate to accelerating suds formation as it forms complex interaction at the interface with other protein and/or carbohydrates.

Ovoglobulin is a lipid binding protein having a molecular weight range ranging from 30,000 to 50,000 Da with isoelectric points ranging 5.5-5.8. It is believed that Ovoglobulin undergo fast structural arrangement at the interface and promote high grease emulsification and suds volume.

The invention is based on the surprising discovery that a "modified" protein, in particular a modified albumin, more particularly a modified chicken egg albumin, even more particularly a modified albumin selected from the group consisting of modified ovalbumin, modified ovomucin, modified ovoglobulin, is able to produce good sudsing profile when formulated into detergent compositions. Given that the 3 proteins have different mechanism to enhance suds performance, it is preferable to use blends or mixtures of the 3 proteins to maximize the enhancement of suds performance in the detergent composition of the invention.

While unmodified proteins are recognized to improve suds stability, their ability to aid in forming high quality suds is greatly affected by their capacity to expose hydrophobic amino acid residues to the interface both kinetically and quantitatively. In practice, un-modified protein have most of their hydrophobic amino-acid residues folded inward of the protein structures, which together with some lack of structural flexibility, e.g.: optionally due to the presence of disulfide bonds, prevents the protein to quickly or efficiently expose its hydrophobic amino-acid residues at the interface. At time, this is also prevented or slowed down by the aggregation of proteins and/or protein sub-units.

The intent of the protein modification is fully linked to the desire to have de-aggregated protein with any of the following attribute. For example, more flexible protein, molten globular or molten folded protein that can achieve more quickly and/or more complete exposure of hydrophobic residues. For example, alternatively partially or fully unfolded, extended protein. For example, alternatively residues from partial hydrolysis of the proteins. For example, alternatively the incorporation of more hydrophobic residues by chemical modification, e.g., esterification.

Unexpectedly, the Applicants found that a "modified" protein, in particular a modified protein selected from the group consisting of modified ovalbumin, modified ovomucin, modified ovoglobulin, and mixtures thereof, is able to produce a more stable hence longer lasting sudsing profile in detergent wash solutions comprising oily and/or greasy soils, compared to the unmodified protein. Not wishing to be bound by theory, the Applicants believe that the increased sudsing benefits are due to differences in amino acid sequences and/or protein structures of the modified versus the unmodified proteins. The modified protein has improved oil and grease affinity hence oil and grease emulsification properties, and as such releasing surfactant molecules otherwise consumed for oil emulsification to continue stabilizing suds throughout the wash process.

Accordingly, a detergent composition of the present invention comprises a modified protein selected from the group consisting of modified ovalbumin, modified ovomucin, modified ovoglobulin, and mixtures thereof. Preferably the modified ovalbumin is derived from an unmodified ovalbumin which has at least at least 50%, preferably at least 60%, preferably at least 70%, preferably at least 80%, preferably at least 85%, preferably at least 90%, preferably at least 95%, preferably at least 98% or even 100% amino acid identity to a native ovalbumin protein (SEQ ID NO: 1). Preferably modified ovomucin is derived from an unmodified ovomucin which has at least 50%, preferably at least 60%, preferably at least 70%, preferably at least 80%, preferably at least 85%, preferably at least 90%, preferably at least 95%, preferably at least 98% or even 100% amino acid identity to at least one native ovomucin protein selected from the group consisting of SEQ ID NO: 2, SEQ ID NO: 4, and SEQ ID NO: 5, preferably SEQ ID NO: 2 or SEQ ID NO: 5. Preferably the modified ovoglobulin is derived from an unmodified ovoglobulin which has at least 50%, preferably at least 60%, preferably at least 70%, preferably at least 80%, preferably at least 85%, preferably at least 90%, preferably at least 95%, preferably at least 98% or even 100% amino acid identity to a native ovoglobulin protein (SEQ ID NO: 3).

For polypeptide sequence comparison the following settings can be used: Alignment algorithm: Needleman and Wunsch, J. Mol. Biol. 1970, 48: 443-453. As a comparison matrix for amino acid similarity the Blosum62 matrix is used (Henikoff S. and Henikoff J.G., P.N.A.S. USA 1992, 89: 10915-10919). The following gap scoring parameters are used: Gap penalty: 12, gap length penalty: 2, no penalty for end gaps. A given sequence is typically compared against the full-length sequence of SEQ ID NO: 1, SEQ ID NO: 2, SEQ ID NO: 3, SEQ ID NO: 4, or SEQ ID NO: 5 to obtain a score.

Preferably the detergent composition of the invention wherein the modified protein is present in an amount of from 0.01 wt% to 5 wt%, preferably from 0.1% to 2.5 wt%, by weight of the composition.

Preferably the detergent composition of the invention comprises a modified protein selected from the group consisting of modified ovalbumin, modified ovomucin, modified ovoglobulin, and mixtures thereof, and a surfactant

system, wherein the modified protein comprises:

a) from 60 wt% to 95 wt%, preferably from 75% to 90 wt%, by weight of the modified protein of the modified ovalbumin;
b) from 1 wt% to 20 wt%, preferably from 2 wt% to 10 wt%, by weight of the modified protein of the modified ovomucin; and
c) from 1 wt% to 20 wt%, preferably from 5 wt% to 15 wt%, by weight of the modified protein of the modified ovoglobulin.

The invention also includes variants in the form of truncated forms derived from a wild-type modified protein, such as a protein with a truncated N-terminus. The modified protein is predicted to include an N-terminal signal peptide that is likely removed upon secretion by the native organisms. The current invention may also include variants without the N-terminal signal peptide. For example, a modified variant protein which corresponds to the sequence of full length wild-type native ovalbumin protein (SEQ ID NO: 1) without the predicted N-terminal signal peptide, is also part of the current invention. Bioinformatic tools, such as for example, signal peptide prediction server SignalP version 4.1 (Petersen TN., Brunak S., von Heijne G. and Nielsen H. (2011). Nature Methods, 8:785-786), can be used to predict the existence and length of such signal peptides.

It is important that variants of modified proteins retain or preferably even improve the ability of the wild-type proteins to adsorb at an interface and to stabilize that interface. Some performance drop in a given property of modified protein variants may of course be tolerated, but the modified protein variants should retain or preferably improve suitable properties for the relevant application for which they are intended. For instance, screening of variants of one of the wild-types can be used to identify whether they retain or even improve appropriate properties.

Suitable examples of modified protein variants are derived from unmodified protein variants which include one conservative substitution in the peptide, such as a conservative substitution in SEQ ID NO: 1, SEQ ID NO: 2, SEQ ID NO: 3, SEQ ID NO: 4, or SEQ ID NO: 5.

Other suitable examples of modified protein variants are derived from unmodified protein variants which include 10 or fewer conservative substitutions in the peptide, such as five or fewer. The modified proteins of the invention may therefore be derived from unmodified protein which include 1, 2, 3, 4, 5, 6, 7, 8, 9, 10 or more conservative substitutions. The modified proteins can be derived from unmodified proteins which can be produced to contain one or more conservative substitutions by manipulating the nucleotide sequence that encodes them using, for example, standard procedures such as site-directed mutagenesis or PCR. Alternatively, the modified proteins can be derived from unmodified proteins which can be produced to contain one or more conservative substitutions by using peptide synthesis methods, for example, as known in the art.

Examples of amino acids which may be substituted for an original amino acid in the unmodified protein and which are regarded as conservative substitutions include: Ser for Ala; Lys for Arg; Gln or His for Asn; Glu for Asp; Asn for Gln; Asp for Glu; Pro for Gly; Asn or Gln for His; Leu or Val for Ile; Ile or Val for Leu; Arg or Gln for Lys; Leu or Ile for Met; Met, Leu or Tyr for Phe; Thr for Ser; Ser for Thr; Tyr for Trp; Trp or Phe for Tyr; and Ile or Leu for Val.

The modified proteins of the invention may be derived from unmodified proteins which comprise variants of SEQ ID NO: 1, SEQ ID NO: 2, SEQ ID NO: 3, SEQ ID NO: 4, or SEQ ID NO: 5, wherein a short amino acid sequence containing two cysteine residues is added at the C-terminus. These cysteine residues can allow the modified protein variants to form multimers (*i.e.,* dimers, tetramers, hexamers and potentially higher order oligomers) in solution due to the formation of disulfide bonds between the cysteine residues of adjacent modified protein variants.

The modified proteins of the invention can also be derived from unmodified proteins which may also cover any fragments of SEQ ID NO: 1, SEQ ID NO: 2, SEQ ID NO: 3, SEQ ID NO: 4, or SEQ ID NO: 5. Preferably the modified protein fragments can adsorb to an interface and stabilize that interface.

Preferably the modified proteins are chemically modified, physically modified or combinations thereof. Accordingly the modified proteins of the invention are chemically modified by a variety of chemical techniques to produce derivatives having essentially the same activity as the unmodified peptides, and optionally having other desirable properties. For example, carboxylic acid groups of the protein, whether carboxyl-terminal or side chain, maybe provided in the form of a salt of a pharmaceutically-acceptable cation or esterified, for example to form a C1-C6 alkyl ester, or converted to an amide, for example of formula $CONR_1R_2$ wherein $R_1$ and $R_2$ are each independently H or C1-C6 alkyl, or combined to form a heterocyclic ring, such as a 5- or 6-membered ring. Amino groups of the peptide, whether amino-terminal or side chain, may be in the form of a pharmaceutically-acceptable acid addition salt, such as the HCl, HBr, acetic, benzoic, toluene sulfonic, maleic, tartaric and other organic salts, or may be modified to C1-C6 alkyl or dialkyl amino or further converted to an amide. Hydroxyl groups of the peptide side chains maybe converted to alkoxy or ester groups, for example C1-C6 alkoxy or C1-C6 alkyl ester, using well-recognized techniques. Phenyl and phenolic rings of the peptide side chains may be substituted with one or more halogen atoms, such as F, Cl, Br or I, or with C1-C6 alkyl, C1-C6 alkoxy, carboxylic acids and esters thereof, or amides of such carboxylic acids. Methylene groups of the peptide side chains can be extended to homologous C2-C4 alkylenes. Thiols can be protected with any one of a number of well-recognized protecting groups, such as acetamide groups.

Those skilled in the art will also recognize methods for introducing cyclic structures into the modified proteins of the present invention to select and provide conformational constraints to the structure that result in enhanced stability.

Preferably the modified protein is partially or completely denatured protein. Preferably the denatured protein has been denatured by:

i) application of heat to the protein at a temperature of from 60°C to 100°C;
ii) pH treatment of the protein to a pH of from 1 to 4 or from 9 to 13;
iii) chemical denaturation of the protein;
iv) subjecting the protein or an unmodified protein to sonication, preferably high frequency sonication, in a range of from 5 kHz to 50 kHz; or
v) combinations of i) to iv).

Preferably the modified protein is derived from partial or complete desugarization of the protein, preferably the desugarization being performed with enzyme, bacteria or yeast, preferably *by S. cerevisiae.*

Preferably the modified protein is derived from partial or complete esterification of said modified protein or an unmodified protein with C1-C12 linear, cyclic or aromatic alcohol.

Preferably the modified protein is a partially hydrolyzed protein, preferably derived from partial hydrolysis of the protein. Preferably the partial hydrolysis being performed enzymatically by protease and the degree of hydrolyzation is 50% or less, preferably 20% or less, more preferably 10% or less, more preferably the protease is papain.

Preferably the pH of the composition is from 7 to 10, preferably from 8.5 to 9, when measured at a 0.12% finished product composition in demineralized water at 20°C.

Surfactant System

While the protein can also be formulated in absence of a surfactant, the detergent composition preferably also comprises a surfactant system. Preferably the detergent composition of the invention comprises from 1% to 60%, preferably from 5% to 50%, more preferably from 8% to 40%, by weight of the total composition of a surfactant system.

The surfactant system of the composition of the present invention comprises an anionic surfactant. Preferably, the surfactant system for the cleaning composition of the present invention comprises from 1% to 40%, preferably 6% to 35%, more preferably 8% to 30% by weight of the total composition of an anionic surfactant. The anionic surfactant can be any anionic cleaning surfactant, preferably selected from sulfate and/or sulfonate anionic surfactants. HLAS (linear alkylbenzene sulfonate) would be the most preferred sulfonate anionic surfactant. Especially preferred anionic surfactant is selected from the group consisting of alkyl sulfate, alkyl alkoxy sufate and mixtures thereof, and preferably wherein the alkyl alkoxy sulfate is an alkyl ethoxy sulfate. Preferred anionic surfactant is a combination of alkyl sulfates and alkyl ethoxy sulfates with a combined average ethoxylation degree of less than 5, preferably less than 3, more preferably less than 2 and more than 0.5 and an average level of branching of from about 5% to about 40%, more preferably from about 10% to 35%, and even more preferably from about 20% to 30%.

The average alkoxylation degree is the mol average alkoxylation degree of all the components of the mixture *(i.e.,* mol average alkoxylation degree) of the anionic surfactant. In the mol average alkoxylation degree calculation the weight of sulfate anionic surfactant components not having alkoxylate groups should also be included.

$$\text{Mol average alkoxylation degree} = (x1 * \text{alkoxylation degree of surfactant } 1 + x2 * \text{alkoxylation degree of surfactant } 2 + \ldots) / (x1 + x2 + \ldots)$$

wherein x1, x2, ... are the number of moles of each sulfate anionic surfactant of the mixture and alkoxylation degree is the number of alkoxy groups in each sulfate anionic surfactant.

The average level of branching is the weight average % of branching and it is defined according to the following formula:

$$\text{Weight average of branching } (\%) = [(x1 * \text{wt\% branched alcohol 1 in alcohol } 1 + x2 * \text{wt\% branched alcohol 2 in alcohol } 2 + \ldots) / (x1 + x2 + \ldots)] * 100$$

wherein x1, x2, ... are the weight in grams of each alcohol in the total alcohol mixture of the alcohols which were used

as starting material for the anionic surfactant for the composition of the invention. In the weight average branching degree calculation the weight of anionic surfactant components not having branched groups should also be included.

Suitable examples of commercially available sulfates include, those based on Neodol alcohols ex the Shell company, Lial - Isalchem and Safol ex the Sasol company, natural alcohols ex The Procter & Gamble Chemicals company. Suitable sulfonate surfactants for use herein include water-soluble salts of C8-C18 alkyl or hydroxyalkyl sulfonates; C11-C18 alkyl benzene sulfonates (LAS), modified alkylbenzene sulfonate (MLAS); methyl ester sulfonate (MES); and alpha-olefin sulfonate (AOS). Those also include the paraffin sulfonates may be monosulfonates and/or disulfonates, obtained by sulfonating paraffins of 10 to 20 carbon atoms. The sulfonate surfactant also include the alkyl glyceryl sulfonate surfactants.

The surfactant system of the composition of the present invention further comprises a primary co-surfactant system, wherein the primary co-surfactant system is preferably selected from the group consisting of amphoteric surfactant, zwitterionic surfactant and mixtures thereof. Preferably, the surfactant system for the cleaning composition of the present invention comprises from 0.5% to 15%, preferably from 1% to 12%, more preferably from 2% to 10%, by weight of the total composition of a primary co-surfactant system.

Preferably the primary co-surfactant system is an amphoteric surfactant. Preferably, the primary co-surfactant system is an amine oxide surfactant, and wherein the composition comprises anionic surfactant and amine oxide surfactant in a ratio of less than 9:1, more preferably from 5:1 to 1:1, more preferably from 4:1 to 2:1, preferably from 3:1 to 2.5:1. Preferred amine oxides are alkyl dimethyl amine oxide or alkyl amido propyl dimethyl amine oxide, more preferably alkyl dimethyl amine oxide and especially coco dimethyl amino oxide. Amine oxide may have a linear or mid-branched alkyl moiety. Typical linear amine oxides include water-soluble amine oxides containing one R1 C8-18 alkyl moiety and 2 R2 and R3 moieties selected from the group consisting of C1-3 alkyl groups and C1-3 hydroxyalkyl groups. Preferably amine oxide is characterized by the formula R1 - N(R2)(R3) O wherein R1 is a C8-18 alkyl and R2 and R3 are selected from the group consisting of methyl, ethyl, propyl, isopropyl, 2-hydroxethyl, 2-hydroxypropyl and 3-hydroxypropyl. The linear amine oxide surfactants in particular may include linear C10-C18 alkyl dimethyl amine oxides and linear C8-C12 alkoxy ethyl dihydroxy ethyl amine oxides. Preferred amine oxides include linear C10, linear C10-C12, and linear C12-C14 alkyl dimethyl amine oxides. As used herein "mid-branched" means that the amine oxide has one alkyl moiety having n1 carbon atoms with one alkyl branch on the alkyl moiety having n2 carbon atoms. The alkyl branch is located on the $\alpha$ carbon from the nitrogen on the alkyl moiety. This type of branching for the amine oxide is also known in the art as an internal amine oxide. The total sum of n1 and n2 is from 10 to 24 carbon atoms, preferably from 12 to 20, and more preferably from 10 to 16. The number of carbon atoms for the one alkyl moiety (n1) should be approximately the same number of carbon atoms as the one alkyl branch (n2) such that the one alkyl moiety and the one alkyl branch are symmetric. As used herein "symmetric" means that | n1 - n2 | is less than or equal to 5, preferably 4, most preferably from 0 to 4 carbon atoms in at least 50 wt%, more preferably at least 75 wt% to 100 wt% of the mid-branched amine oxides for use herein. The amine oxide further comprises two moieties, independently selected from a C1-3 alkyl, a C1-3 hydroxyalkyl group, or a polyethylene oxide group containing an average of from about 1 to about 3 ethylene oxide groups. Preferably, the two moieties are selected from a C1-3 alkyl, more preferably both are selected as a C1 alkyl.

Preferably the amine oxide surfactant is a mixture of amine oxides comprising a low-cut amine oxide and a mid-cut amine oxide. The amine oxide of the composition of the invention then comprises:

a) from about 10% to about 45% by weight of the amine oxide of low-cut amine oxide of formula R1R2R3AO wherein R1 and R2 are independently selected from hydrogen, C1-C4 alkyls or mixtures thereof, and R3 is selected from C10 alkyls or mixtures thereof; and

b) from 55% to 90% by weight of the amine oxide of mid-cut amine oxide of formula R4R5R6AO wherein R4 and R5 are independently selected from hydrogen, C1-C4 alkyls or mixtures thereof, and R6 is selected from C12-C16 alkyls or mixtures thereof

In a preferred low-cut amine oxide for use herein R3 is n-decyl. In another preferred low-cut amine oxide for use herein R1 and R2 are both methyl. In an especially preferred low-cut amine oxide for use herein R1 and R2 are both methyl and R3 is n-decyl.

Preferably, the amine oxide comprises less than about 5%, more preferably less than 3%, by weight of the amine oxide of an amine oxide of formula R7R8R9AO wherein R7 and R8 are selected from hydrogen, C1-C4 alkyls and mixtures thereof and wherein R9 is selected from C8 alkyls and mixtures thereof. Compositions comprising R7R8R9AO tend to be unstable and do not provide very suds mileage.

Preferably the primary co-surfactant system is a zwitterionic surfactant. Suitable exampes of zwitterionic surfactants include betaines, such as alkyl betaines, alkylamidobetaine, amidazoliniumbetaine, sulfobetaine (INCI Sultaines) as well as the Phosphobetaine and preferably meets formula (I):

R1-[CO-X (CH2)n]x-N+(R2)(R3)-(CH2)m-[CH(OH)-CH2]y-Y- (I)

wherein

R1 is a saturated or unsaturated C6-22 alkyl residue, preferably C8-18 alkyl residue, in particular a saturated C10-16 alkyl residue, for example a saturated C12-14 alkyl residue;
X is NH, NR4 with C1-4 Alkyl residue R4, O or S;
n a number from 1 to 10, preferably 2 to 5, in particular 3;
x is 0 or 1, preferably 1;
R2, R3 are independently a C1-4 alkyl residue, potentially hydroxy substituted such as a hydroxyethyl, preferably a methyl;
m a number from 1 to 4, in particular 1, 2 or 3;
y 0 or 1; and
Y is COO, SO3, OPO(OR5)O or P(O)(OR5)O, whereby R5 is a hydrogen atom H or a C1-4 alkyl residue.

Preferred betaines are the alkyl betaines of the formula (Ia), the alkyl amido propyl betaine of the formula (Ib), the Sulfo betaines of the formula (Ic), and the Amido sulfobetaine of the formula (Id);

R1-N+(CH3)2-CH2COO- (Ia)

R1-CO-NH(CH2)3-N+(CH3)2-CH2COO- (Ib)

R1-N+(CH3)2-CH2CH(OH)CH2SO3- (Ic)

R1-CO-NH-(CH2)3-N+(CH3)2-CH2CH(OH)CH2SO3- (Id)

in which R11 as the same meaning as in formula I. Particularly preferred betaines are the Carbobetaine [wherein Y-=COO-], in particular the Carbobetaine of the formula (Ia) and (Ib), more preferred are the Alkylamidobetaine of the formula (Ib).

Examples of suitable betaines and sulfobetaine are the following [designated in accordance with INCI]: Almondamidopropyl of betaines, Apricotam idopropyl betaines, Avocadamidopropyl of betaines, Babassuamidopropyl of betaines, Behenam idopropyl betaines, Behenyl of betaines, betaines, Canolam idopropyl betaines, Capryl/Capram idopropyl betaines, Carnitine, Cetyl of betaines, Cocamidoethyl of betaines, Cocam idopropyl betaines, Cocam idopropyl Hydroxysultaine, Coco betaines, Coco Hydroxysultaine, Coco/Oleam idopropyl betaines, Coco Sultaine, Decyl of betaines, Dihydroxyethyl Oleyl Glycinate, Dihydroxyethyl Soy Glycinate, Dihydroxyethyl Stearyl Glycinate, Dihydroxyethyl Tallow Glycinate, Dimethicone Propyl of PG-betaines, Erucam idopropyl Hydroxysultaine, Hydrogenated Tallow of betaines, Isostearam idopropyl betaines, Lauram idopropyl betaines, Lauryl of betaines, Lauryl Hydroxysultaine, Lauryl Sultaine, Milkam idopropyl betaines, Minkamidopropyl of betaines, Myristam idopropyl betaines, Myristyl of betaines, Oleam idopropyl betaines, Oleam idopropyl Hydroxysultaine, Oleyl of betaines, Olivamidopropyl of betaines, Palmam idopropyl betaines, Palm itam idopropyl betaines, Palmitoyl Carnitine, Palm Kernelam idopropyl betaines, Polytetrafluoroethylene Acetoxypropyl of betaines, Ricinoleam idopropyl betaines, Sesam idopropyl betaines, Soyam idopropyl betaines, Stearam idopropyl betaines, Stearyl of betaines, Tallowam idopropyl betaines, Tallowam idopropyl Hydroxysultaine, Tallow of betaines, Tallow Dihydroxyethyl of betaines, Undecylenam idopropyl betaines and Wheat Germam idopropyl betaines. A preferred betaine is, for example, Cocoamidopropylbetaine.

Preferably the surfactant system of the composition of the present invention further comprises from 0.1% to 10% by weight of the total composition of a secondary co-surfactant system preferably comprising a non-ionic surfactant. Suitable non-ionic surfactants include the condensation products of aliphatic alcohols with from 1 to 25 moles of ethylene oxide. The alkyl chain of the aliphatic alcohol can either be straight or branched, primary or secondary, and generally contains from 8 to 22 carbon atoms. Particularly preferred are the condensation products of alcohols having an alkyl group containing from 10 to 18 carbon atoms, preferably from 10 to 15 carbon atoms with from 2 to 18 moles, preferably 2 to 15, more preferably 5-12 of ethylene oxide per mole of alcohol. Highly preferred non-ionic surfactants are the condensation products of guerbet alcohols with from 2 to 18 moles, preferably 2 to 15, more preferably 5-12 of ethylene oxide per mole of alcohol. Preferably, the non-ionic surfactants are an alkyl ethoxylated surfactants, preferably comprising from 9 to 15 carbon atoms in its alkyl chain and from 5 to 12 units of ethylene oxide per mole of alcohol. Other suitable non-ionic surfactants for use herein include fatty alcohol polyglycol ethers, alkylpolyglucosides and fatty acid glucamides, preferably alkylpolyglucosides. Preferably the alkyl polyglucoside surfactant is a C8-C16 alkyl polyglucoside surfactant, preferably a C8-C14 alkyl polyglucoside surfactant, preferably with an average degree of polymerization of between 0.1 and 3, more preferably between 0.5 and 2.5, even more preferably between 1 and 2. Most preferably the alkyl polyglucoside surfactant has an average alkyl carbon chain length between 10 and 16, preferably between 10 and 14, most

preferably between 12 and 14, with an average degree of polymerization of between 0.5 and 2.5 preferably between 1 and 2, most preferably between 1.2 and 1.6. C8-C16 alkyl polyglucosides are commercially available from several suppliers (e.g., Simusol® surfactants from Seppic Corporation; and Glucopon® 600 CSUP, Glucopon® 650 EC, Glucopon® 600 CSUP/MB, and Glucopon® 650 EC/MB, from BASF Corporation). Preferably, the composition comprises the anionic surfactant and the non-ionic surfactant in a ratio of from 2:1 to 50:1, preferably 2:1 to 10:1.

Enzymes

Preferred compositions of the invention may comprise one or more enzymes selected from the group consisting of amylases, lipases, proteases, cellulases, lipoxygenases, diol synthases, and mixtures thereof. Each additional enzyme is typically present in an amount from 0.0001 wt% to 1 wt% (by weight of active protein) more preferably from 0.0005 wt% to 0.5 wt%, most preferably 0.005 wt% to 0.1 wt%.

Enzyme Stabilizer

When comprising enzymes preferably the composition of the invention comprises an enzyme stabilizer. Suitable enzyme stabilizers may be selected from the group consisting of (a) univalent, bivalent and/or trivalent cations preferably selected from the group of inorganic or organic salts of alkaline earth metals, alkali metals, aluminum, iron, copper and zinc, preferably alkali metals and alkaline earth metals, preferably alkali metal and alkaline earth metal salts with halides, sulfates, sulfites, carbonates, hydrogencarbonates, nitrates, nitrites, phosphates, formates, acetates, propionates, citrates, maleates, tartrates, succinates, oxalates, lactates, and mixtures thereof. In a preferred embodiment the salt is selected from the group consisting of sodium chloride, calcium chloride, potassium chloride, sodium sulfate, potassium sulfate, sodium acetate, potassium acetate, sodium formate, potassium formate, calcium lactate, calcium nitrate and mixtures thereof. Most preferred are salts selected from the group consisting of calcium chloride, potassium chloride, potassium sulfate, sodium acetate, potassium acetate, sodium formate, potassium formate, calcium lactate, calcium nitrate, and mixtures thereof, and in particular potassium salts selected from the group of potassium chloride, potassium sulfate, potassium acetate, potassium formate, potassium propionate, potassium lactate and mixtures thereof. Most preferred are potassium acetate and potassium chloride. Preferred calcium salts are calcium formate, calcium lactate and calcium nitrate including calcium nitrate tetrahydrate. Calcium and sodium formate salts may be preferred. These cations are present at at least 0.01 wt%, preferably at least 0.03 wt%, more preferably at least 0.05 wt%, most preferably at least 0.25 wt% up to 2 wt% or even up to 1 wt% by weight of the total composition. These salts are formulated from 0.1 to 5 wt%, preferably from 0.2 to 4 wt%, more preferably from 0.3 to 3 wt%, most preferably from 0.5 to 2 wt% relative to the total weight of the composition. Further enzyme stabilizers can be selected from the group (b) carbohydrates selected from the group consisting of oligosaccharides, polysaccharides and mixtures thereof, such as a monosaccharide glycerate as described in WO201219844; (c) mass efficient reversible protease inhibitors selected from the group consisting of phenyl boronic acid and derivatives thereof, preferably 4-formyl phenylboronic acid; (d) alcohols such as 1,2-propane diol, propylene glycol; (e) peptide aldehyde stabilizers such as tripeptide aldehydes such as Cbz-Gly-Ala-Tyr-H, or disubstituted alaninamide; (f) carboxylic acids such as phenyl alkyl dicarboxylic acid as described in WO2012/19849 or multiply substituted benzyl carboxylic acid comprising a carboxyl group on at least two carbon atoms of the benzyl radical such as described in WO2012/19848, phthaloyl glutamine acid, phthaloyl asparagine acid, aminophthalic acid and/or an oligoamino-biphenyl-oligocarboxylic acid; and (g) mixtures thereof.

Salt

The composition of the present invention may optionally comprise from 0.01% to 3%, preferably from 0.05% to 2%, more preferably from 0.2% to 1.5%, or most preferably 0.5% to 1%, by weight of the total composition of a salt, preferably a monovalent, divalent inorganic salt or a mixture thereof, preferably sodium chloride. Most preferably the composition alternatively or further comprises a multivalent metal cation in the amount of from 0.01 wt% to 2 wt%, preferably from 0.1% to 1%, more preferably from 0.2% to 0.8% by weight of the composition, preferably the multivalent metal cation is magnesium, aluminium, copper, calcium or iron, more preferably magnesium, most preferably said multivalent salt is magnesium chloride. Without wishing to be bound by theory, it is believed that use of a multivalent cation helps with the formation of protein/ protein, surfactant/ surfactant or hybrid protein/ surfactant network at the oil water and air water interface that is strengthening the suds.

Carbohydrates

Preferably the composition of the present invention comprises one or more carbohydrates selected from the group comprising O-glycan, N-glycan, and mixtures thereof. Suitable carbohydrates include alpha or beta glucan with

1,3 and/or 1.4 and/or 1,6 linkage. Glucans can be modified especially with carboxyl sulfate, glycol ether of amino groups. Glucan can be extracted from dextran, starch or cellulose. Glucan with structure close to natural glucan such as schizophyllan, scleroglucan or paramylon are particularly preferred.

Hydrotrope

The composition of the present invention may optionally comprise from 1% to 10%, or preferably from 0.5% to 10%, more preferably from 1% to 6%, or most preferably from 0.1% to 3%, or combinations thereof, by weight of the total composition of a hydrotrope, preferably sodium cumene sulfonate. Other suitable hydrotropes for use herein include anionic-type hydrotropes, particularly sodium, potassium, and ammonium xylene sulfonate, sodium, potassium and ammonium toluene sulfonate, sodium potassium and ammonium cumene sulfonate, and mixtures thereof, as disclosed in U.S. Patent 3,915,903. Preferably the composition of the present invention is isotropic. An isotropic composition is distinguished from oil-in-water emulsions and lamellar phase compositions. Polarized light microscopy can assess whether the composition is isotropic. See e.g., The Aqueous Phase Behaviour of Surfactants, Robert Laughlin, Academic Press, 1994, pp. 538-542. Preferably an isotropic composition is provided. Preferably the composition comprises 0.1% to 3% by weight of the total composition of a hydrotrope, preferably wherein the hydrotrope is selected from sodium, potassium, and ammonium xylene sulfonate, sodium, potassium and ammonium toluene sulfonate, sodium potassium and ammonium cumene sulfonate, and mixtures thereof.

Organic solvent

The composition of the present invention may optionally comprise an organic solvent. Suitable organic solvents include C4-14 ethers and diethers, polyols, glycols, alkoxylated glycols, C6-C16 glycol ethers, alkoxylated aromatic alcohols, aromatic alcohols, aliphatic linear or branched alcohols, alkoxylated aliphatic linear or branched alcohols, alkoxylated C1-C5 alcohols, C8-C14 alkyl and cycloalkyl hydrocarbons and halohydrocarbons, and mixtures thereof. Preferably the organic solvents include alcohols, glycols, and glycol ethers, alternatively alcohols and glycols. The composition comprises from 0% to less than 50%, preferably from 0.01% to 25%, more preferably from 0.1% to 10%, or most preferably from 0.5% to 5%, by weight of the total composition of an organic solvent, preferably an alcohol, more preferably an ethanol, a polyalkyleneglycol, more preferably polypropyleneglycol, and mixtures thereof.

Amphiphilic Polymer

The composition of the present invention may further comprise from about 0.01% to about 5%, preferably from about 0.05% to about 2%, more preferably from about 0.07% to about 1% by weight of the total composition of an amphiphilic polymer selected from the groups consisting of amphiphilic alkoxylated polyalkyleneimine and mixtures thereof, preferably an amphiphilic alkoxylated polyalkyleneimine.

Preferably, the amphiphilic alkoxylated polyalkyleneimine is an alkoxylated polyethyleneimine polymer comprising a polyethyleneimine backbone having average molecular weight range from 100 to 5,000, preferably from 400 to 2,000, more preferably from 400 to 1,000 Daltons and the alkoxylated polyethyleneimine polymer further comprising:

(i) one or two alkoxylation modifications per nitrogen atom by a polyalkoxylene chain having an average of about 1 to about 50 alkoxy moieties per modification, wherein the terminal alkoxy moiety of the alkoxylation modification is capped with hydrogen, a C1-C4 alkyl or mixtures thereof;
(ii) an addition of one C1-C4 alkyl moiety and one or two alkoxylation modifications per nitrogen atom by a polyalkoxylene chain having an average of about 1 to about 50 alkoxy moieties per modification wherein the terminal alkoxy moiety is capped with hydrogen, a C1-C4 alkyl or mixtures thereof; or
(iii)a combination thereof; and

wherein the alkoxy moieties comprises ethoxy (EO) and/or propxy (PO) and/or butoxy (BO) and wherein when the alkoxylation modification comprises EO it also comprises PO or BO.

Preferred amphiphilic alkoxylated polyethyleneimine polymers comprise EO and PO groups within their alkoxylation chains, the PO groups preferably being in terminal position of the alkoxy chains, and the alkoxylation chains preferably being hydrogen capped. Hydrophilic alkoxylated polyethyleneimine polymers solely comprising ethoxy (EO) units within the alkoxylation chain could also optionally be formulated within the scope of this invention.

For example, but not limited to, below is shown possible modifications to terminal nitrogen atoms in the polyethyleneimine backbone where R represents an ethylene spacer and E represents a C1-C4 alkyl moiety and X- represents a suitable water soluble counterion.

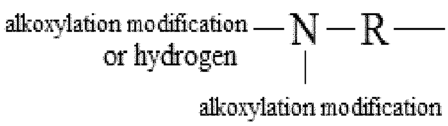

or

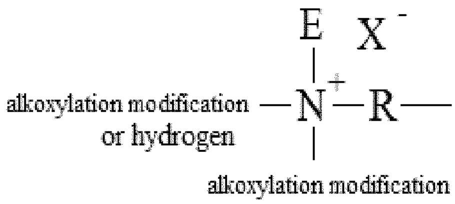

Also, for example, but not limited to, below is shown possible modifications to internal nitrogenatoms in the polyethyleneimine backbone where R represents an ethylene spacer and E represents a $C_1$-$C_4$ alkyl moiety and X- represents a suitable water soluble counterion.

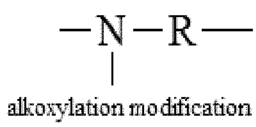

or

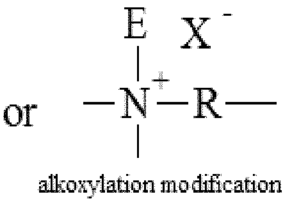

The alkoxylation modification of the polyethyleneimine backbone consists of the replacement of a hydrogen atom by a polyalkoxylene chain having an average of about 1 to about 50 alkoxy moieties, preferably from about 20 to about 45 alkoxy moieties, most preferably from about 30 to about 45 alkoxy moieties. The alkoxy moieties are selected from ethoxy (EO), propoxy (PO),butoxy (BO), and mixtures thereof. Alkoxy moieties solely comprising ethoxy units are outside the scope of the invention though. Preferably, the polyalkoxylene chain is selected from ethoxy/propoxy block moieties. More preferably, the polyalkoxylene chain is ethoxy/propoxy block moieties having an average degree of ethoxylation from about 3 to about 30 and an average degree of propoxylation from about 1 to about 20, more preferably ethoxy/propoxy block moieties having an average degree of ethoxylation from about 20 to about 30 and an average degree of propoxylation from about 10 to about 20.

More preferably the ethoxy/propoxy block moieties have a relative ethoxy to propoxy unit ratio between 3 to 1 and 1 to 1, preferably between 2 to 1 and 1 to 1. Most preferably the polyalkoxylene chain is the ethoxy/propoxy block moieties wherein the propoxy moiety block is the terminal alkoxy moiety block.

The modification may result in permanent quaternization of the polyethyleneimine backbone nitrogen atoms. The degree of permanent quaternization may be from 0% to about 30% of the polyethyleneimine backbone nitrogen atoms. It is preferred to have less than 30% of the polyethyleneimine backbone nitrogen atoms permanently quaternized. Most preferably the degree of quaternization is about 0%.

A preferred polyethyleneimine has the general structure of Formula (II):

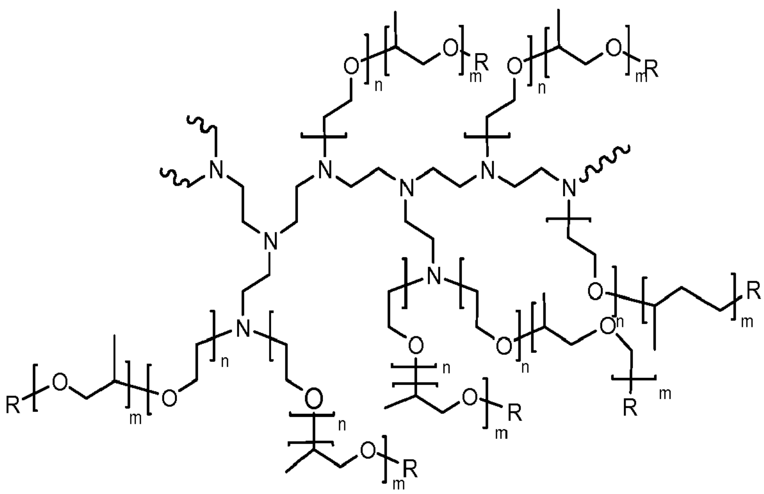

(II)

wherein the polyethyleneimine backbone has a weight average molecular weight of about 600, n of formula (II) has an average of about 10, m of formula (II) has an average of about 7 and R of formula (II) is selected from hydrogen, a $C_1$-$C_4$

alkyl and mixtures thereof, preferably hydrogen. The degree of permanent quaternization of formula (II) may be from 0% to about 22% of the polyethyleneimine backbone nitrogen atoms. The molecular weight of this polyethyleneimine preferably is between 10,000 and 15,000.

An alternative polyethyleneimine has the general structure of Formula (II) but wherein the polyethyleneimine backbone has a weight average molecular weight of about 600, n of Formula (II) has an average of about 24, m of Formula (II) has an average of about 16 and R of Formula (II) is selected from hydrogen, a $C_1$-$C_4$ alkyl and mixtures thereof, preferably hydrogen. The degree of permanent quaternization of Formula (II) may be from 0% to about 22% of the polyethyleneimine backbone nitrogen atoms. The molecular weight of this polyethyleneimine preferably is between 25,000 and 30,000.

Most preferred polyethyleneimine has the general structure of Formula (II) wherein the polyethyleneimine backbone has a weight average molecular weight of about 600, n of Formula (II) has an average of about 24, m of Formula (II) has an average of about 16 and R of Formula (II) is hydrogen. The degree of permanent quaternization of Formula (II) is 0% of the polyethyleneimine backbone nitrogen atoms. The molecular weight of this polyethyleneimine preferably is about from about 25,000 to 30,000, most preferably about 28,000.

These polyethyleneimines can be prepared, for example, by polymerizing ethyleneimine in the presence of a catalyst such as carbon dioxide, sodium bisulfite, sulfuric acid, hydrogen peroxide, hydrochloric acid, acetic acid, and the like, as described in more detail in PCT Publication No. WO 2007/135645.

## Chelant

The detergent composition herein can comprise a chelant at a level of from 0.1% to 20%, preferably from 0.2% to 5%, more preferably from 0.2% to 3% by weight of total composition.

As commonly understood in the detergent field, chelation herein means the binding or complexation of a bi- or multidentate ligand. These ligands, which are often organic compounds, are called chelants, chelators, chelating agents, and/or sequestering agent. Chelating agents form multiple bonds with a single metal ion. Chelants, are chemicals that form soluble, complex molecules with certain metal ions, inactivating the ions so that they cannot normally react with other elements or ions to produce precipitates or scale, or forming encrustations on soils turning them harder to be removed. The ligand forms a chelate complex with the substrate. The term is reserved for complexes in which the metal ion is bound to two or more atoms of the chelant.

Preferably, the composition of the present invention comprises one or more chelant, preferably selected from the group comprising carboxylate chelants, amino carboxylate chelants, amino phosphonate chelants such as MGDA (methylglycine-N,N-diacetic acid), GLDA (glutamic-N,N- diacetic acid), and mixtures thereof.

Suitable chelating agents can be selected from the group consisting of amino carboxylates, amino phosphonates, polycarboxylate chelating agents and mixtures thereof.

Other chelants include homopolymers and copolymers of polycarboxylic acids and their partially or completely neutralized salts, monomeric polycarboxylic acids and hydroxycarboxylic acids and their salts. Suitable polycarboxylic acids are acyclic, alicyclic, heterocyclic and aromatic carboxylic acids, in which case they contain at least two carboxyl groups which are in each case separated from one another by, preferably, no more than two carbon atoms. A suitable hydroxycarboxylic acid is, for example, citric acid. Another suitable polycarboxylic acid is the homopolymer of acrylic acid. Preferred are the polycarboxylates end capped with sulfonates.

## Adjunct Ingredients

The cleaning composition herein may optionally comprise a number of other adjunct ingredients such as builders (e.g., preferably citrate), cleaning solvents, cleaning amines, conditioning polymers, cleaning polymers, surface modifying polymers, soil flocculating polymers, structurants, emollients, humectants, skin rejuvenating actives, enzymes, carboxylic acids, scrubbing particles, bleach and bleach activators, perfumes, malodor control agents, pigments, dyes, opacifiers, beads, pearlescent particles, microcapsules, inorganic cations such as alkaline earth metals such as Ca/Mg-ions, antibacterial agents, preservatives, viscosity adjusters (*e.g.,* salt such as NaCl, and other mono-, di- and trivalent salts) and pH adjusters and buffering means (e.g., carboxylic acids such as citric acid, HCl, NaOH, KOH, alkanolamines, phosphoric and sulfonic acids, carbonates such as sodium carbonates, bicarbonates, sesquicarbonates, borates, silicates, phosphates, imidazole and alike).

## Method of Washing

In another aspect, the invention is directed to a method of manually washing dishware comprising the steps of delivering a detergent composition of the invention into a volume of water to form a wash solution and immersing the dishware in the solution. As such, the composition herein will be applied in its diluted form to the dishware. Soiled surfaces

e.g. dishes are contacted with an effective amount, typically from 0.5 mL to 20 mL (per 25 dishes being treated), preferably from 3mL to 10 mL, of the detergent composition of the present invention, preferably in liquid form, diluted in water. The actual amount of detergent composition used will be based on the judgment of user, and will typically depend upon factors such as the particular product formulation of the composition, including the concentration of active ingredients in the composition, the number of soiled dishes to be cleaned, the degree of soiling on the dishes, and the like. Generally, from 0.01 mL to 150 mL, preferably from 3 mL to 40 mL of a liquid detergent composition of the invention is combined with from 2,000 mL to 20,000 mL, more typically from 5,000 mL to 15,000 mL of water in a sink having a volumetric capacity in the range of from 1,000 mL to 20,000 mL, more typically from 5,000 mL to 15,000 mL. The soiled dishes are immersed in the sink containing the diluted compositions then obtained, where contacting the soiled surface of the dish with a cloth, sponge, or similar article cleans them. The cloth, sponge, or similar article may be immersed in the detergent composition and water mixture prior to being contacted with the dish surface, and is typically contacted with the dish surface for a period of time ranged from 1 to 10 seconds, although the actual time will vary with each application and user. The contacting of cloth, sponge, or similar article to the surface is preferably accompanied by a concurrent scrubbing of the surface.

In another aspect, the invention is directed to a method of manually washing dishware with the composition of the present invention. The method comprises the steps of: i) delivering a composition of the present invention onto the dishware or a cleaning implement; ii) cleaning the dishware with the composition in the presence of water; and iii) optionally, rinsing the dishware. The delivering step is preferably either directly onto the dishware surface or onto a cleaning implement, *i.e.,* in a neat form. The cleaning device or implement is preferably wet before or after the composition is delivered to it. Especially good grease removal has been found when the composition is used in neat form.

In another aspect, the invention is directed to a method of manually washing soiled articles comprising contacting a detergent composition with a surface, wherein the composition comprises a modified protein selected from the group consisting of modified ovalbumin, modified ovomucin, modified ovoglobulin, and mixtures thereof and a surfactant system, and wherein the composition modifies the hydrophobicity of the surface as a result of the contacting step.

Another aspect of the present invention is directed to a method of promoting suds longevity or grease emulsification in a washing process for washing soiled articles, preferably dishware or fabric. The method comprises the steps of: a) delivering a detergent composition comprising a modified protein selected from the group consisting of modified ovalbumin, modified ovomucin, modified ovoglobulin, and mixtures thereof and a surfactant system to a volume of water to form a wash liquor; and b) immersing the soiled articles into said wash liquor. Preferably, the modified protein is present at a concentration of about 0.005 ppm to about 60 ppm, preferably at a concentration of about 0.02 ppm to about 12 ppm, in an aqueous wash liquor during the washing process.

Another aspect of the present invention is use of a modified protein selected from the group consisting of modified ovalbumin, modified ovomucin, modified ovoglobulin, and mixtures thereof to provide increased suds longevity or increased grease emulsification in an aqueous wash liquor during a washing process. Preferably the modified ovalbumin is derived from an unmodified ovalbumin which has at least at least 50%, preferably at least 60%, preferably at least 70%, preferably at least 80%, preferably at least 85%, preferably at least 90%, preferably at least 95%, preferably at least 98% or even 100% amino acid identity to a native ovalbumin protein (SEQ ID NO: 1). Preferably the modified ovomucin is derived from an unmodified ovomucin has at least 50%, preferably at least 60%, preferably at least 70%, preferably at least 80%, preferably at least 85%, preferably at least 90%, preferably at least 95%, preferably at least 98% or even 100% amino acid identity to at least one native ovomucin protein selected from the group consisting of SEQ ID NO: 2, SEQ ID NO: 4, and SEQ ID NO: 5, preferably SEQ ID NO: 2 or SEQ ID NO: 5. Preferably the modified ovoglobulin is derived from an unmodified ovoglobulin which has at least 50%, preferably at least 60%, preferably at least 70%, preferably at least 80%, preferably at least 85%, preferably at least 90%, preferably at least 95%, preferably at least 98% or even 100% amino acid identity to a native ovoglobulin protein (SEQ ID NO: 3).

## TEST METHODS

The following assays set forth must be used in order that the invention described and claimed herein may be more fully understood.

### Test Method 1 - Suds Mileage

The evolution of the suds volume generated by a certain solution of dishwashing liquid is followed at 15 dh hardness, at solution temperature of 46°C, and detergent concentrations (0.12 wt%), under influence of periodic soil injections. Data are compared and expressed versus a reference product as a suds mileage index (reference product has suds mileage index of 100).

A defined amount of dishwashing product depending on the targeted detergent concentration (0.12 wt%) is dispensed through a pipette with a flow rate of 0.67 mL/ sec at a height of 37 cm above the sink bottom surface into a

water stream that starts filling up a sink (dimensions : cylinder - diameter 300 mm & height 288 mm) to 4 L with a constant pressure of 4 bar. With this pressure an initial suds volume is generated in the sink.

After recording the initial suds volume (average suds height * sink surface area) a fixed amount of greasy soil (6 mL) according to Table 1 will be injected almost instantaneously in the middle of the sink, while a paddle (metal blade 10 cm x 5 cm, positioned in the middle of the sink at the air liquid interface under an angle of 45 degrees) will rotate 20 times into the solution at 85 RPM. This step is followed immediately by another measurement of the total suds volume. The soil injecting, paddling and measuring steps are repeated until the measured suds volume reaches a minimum level, which is set at 400 $cm^3$. The amount of soil additions needed to get to that level is considered as the mileage of that specific sample.

The complete process is repeated 4 times per sample and per testing condition (temperature, concentration, hardness, soil type). As a final result the average mileage of the 4 replicates is calculated for each sample per soil type and averaged across testing conditions. Comparing the average mileage of the test sample versus that of the reference sample, indicates the performance of the test sample versus the reference sample, and is expressed as a suds mileage index, calculated as (average number of soil additions of test sample / average number of soil additions of reference sample) *100.

Table 1: Greasy Soil Composition

| **Ingredient** | **Weight %** |
|---|---|
| Crisco oil | 12.730 |
| Crisco shortening | 27.752 |
| Lard | 7.638 |
| Refined Rendered Edible Beef Tallow | 51.684 |
| Oleic Acid, 90% (Techn) | 0.139 |
| Palmitic Acid, 99+% | 0.036 |
| Stearic Acid, 99+% | 0.021 |

EXAMPLES

The following examples are provided to further illustrate the present invention and are not to be construed as limitations of the present invention, as many variations of the present invention are possible without departing from its spirit or scope.

Example 1 - Protein Modifications

The proteins of the invention are modified separately or as a blend of proteins. The proteins are modified according to the processes described in Table 2, or a mixture thereof.

Table 2: Modifications

| | | |
|---|---|---|
| 1 | Desugarization | Desugarization is achieved by treatment of food-grade baker's yeast (*Saccharomyces cerevisiae*) for 3 hrs at 30°C, and adjustment of the pH to 7 with citric or lactic acid. Desugarization can also occur by other bacterial treatment (*e.g.,* with *lactobacillus brevis, lactobacillus casei, streptococcus diacetilactis,* or *klebsiella Pneumoniae*) or by enzymatic treatment (e.g., Glucose oxidase (EC 1.13.4)) with or without catalase (EC 1.11.1.6) and with or without $H_2O_2$. |
| 2 | Denaturation by heat | Dried proteins with remaining water content below 10% are submitted to a thermal treatment at 80°C for 96 hrs. |
| 3 | Denaturation by pH | Proteins are subjected to extreme pH to promote unfolding and expose hydrophobic residues to the protein surface. Preferred pH treatment is performed with 1N NaOH to adjust the pH to 10-13, preferably 12 for at least 5 minutes at 20°C before formulating into the detergent composition. Alternatively acidic pH treatment is performed with 1N HCl to adjust the pH to 1-4, preferably 2 for at least 5 minutes at 20°C before formulating into the detergent composition. |

(continued)

| | | |
|---|---|---|
| 4 | Denaturation by sonication | A 10% protein solution is adjusted to pH 8 and sonicated with a VWR sonicator at 35kHz for 3 hrs at 20°C. The pH of the sonicated protein solution is adjusted to pH 9 prior to usage. |
| 5 | Chemical Denaturation | A 10% protein solution is treated with 8M Urea and 1M $Na_2SO_3$ at 38°C for 6 hrs. The solution is dialyzed with 1% AES surfactant at pH 9 prior to usage. |
| 6 | Chemical modification | A 10% protein solution is esterified with 5% phenyl chloroformate at 20°C for 1 hr. The pH of the solution is adjusted continually during the reaction with 1N NaOH to pH 4. At the end of the reaction, the pH is restored to pH 9, prior to usage. |
| 7 | Hydrolysis | A 10% protein solution is adjusted with 1N NaOH to pH 7 and treated with 0.3% papain enzyme for 1hr at 35°C. Other suitable enzymes include ficin, trypsin, or bromelain. |

Example 2 - Suds Mileage Performance

The ability to maintain suds mileage is assessed for detergent compositions comprising modified proteins according to Table 3. The foregoing compositions are produced through standard mixing of the components described in Table 3.

Table 3 - Exemplary Detergent Compositions**

| **Ingredients** | **Ex. 1** | **Ex. 2** | **Ex. 3** | **Ex. 4** | **Ex. 5** |
|---|---|---|---|---|---|
| Sodium alkyl ethoxy sulfate (C1213EO0.6S) | 22.91% | 22.91% | 22.91% | 22.91% | 22.91% |
| n-C12-14 Di Methyl Amine Oxide | 7.64% | 7.64% | 7.64% | 7.64% | 7.64% |
| Lutensol® XP80 (non-ionic surfactant supplied by BASF) | 0.45% | 0.45% | 0.45% | 0.45% | 0.45% |
| Sodium Chloride | 1.2% | 1.2% | 1.2% | 1.2% | 1.2% |
| Poly Propylene Glycol | 1% | 1% | 1% | 1% | 1% |
| Ethanol | 2% | 2% | 2% | 2% | 2% |
| Sodium Hydroxide | 0.24% | 0.24% | 0.24% | 0.24% | 0.24% |
| Ovalbumin* | 0.06% | 1.16% | 1.16% | 1.16% | 1.16% |
| Mucin* | 0.04% | 0.07% | 0.07% | 0.07% | 0.07% |
| Ovoglobulin* | 0.09% | 0.17% | 0.17% | 0.17% | 0.17% |
| Transferrin* | 0.13% | 0.26% | 0.26% | 0.26% | 0.26% |
| Mucoid* | 0.11% | 0.23% | 0.23% | 0.23% | 0.23% |
| Lysozyme* | 0.04% | 0.07% | 0.07% | 0.07% | 0.07% |
| Avidin* | 0.005% | 0.01% | 0.01% | 0.01% | 0.01% |
| Minors (perfume, preservative, dye) + water | To 100 % | To 100 % | To 100 % | To 100 % | To 100 % |
| pH (@ 0.12% solution) | 8.35 | 7.5 | 8.35 | 9 | 10 |

*Note: proteins from egg white and the proteins have been modified according to the invention.
**Comparative Examples 1 to 5: Examples 1 to 5 excluding the egg white proteins and replaced with water.

The resultant compositions are assessed according to the Suds Mileage Index test method as described herein. The suds mileage results are summarized in Table 4. The higher the Suds Mileage Index value, the better in maintaining suds mileage. From the data it can be concluded that the Inventive Compositions Ex. 1 & 3 comprising modified proteins according to the invention, wherein the modification is desugarization as described in Table 1, have a stronger suds robustness in presence of greasy soils as compared to Comparative Compositions Exs. 1 & 3 outside the scope of the invention *(i.e.,* comprising the native unmodified proteins).

Table 4 - Suds Mileage Index Results of Inventive and Comparative Compositions

| | **Inventive Comp. Ex. 1** | **Inventive Comp. Ex. 3** | **Comparative Comp. Ex. 1** | **Comparative Comp. Ex. 3** |
|---|---|---|---|---|
| Suds Mileage Index (Greasy Soil) | 109 | 115 | 100 | 100 |

Further suds mileage results across wash pH are summarized in Table 5. From the data it can be concluded that Inventive Compositions 2 to 5 comprising modified proteins according to the invention, wherein the modification is desugarization as described in Table 1, have a stronger suds robustness in presence of greasy soils as compared to the nil protein reference at pH 8.35 (@ 0.12% solution - index 100), showing an optimal wash pH between 8 and 9 (@ 0.12% solution).

Table 5 - Suds Mileage Index Results of Inventive and Comparative Compositions Across Wash pH

| | **Inventive Comp. Ex. 2** | **Inventive Comp. Ex. 3** | **Inventive Comp. Ex. 4** | **Inventive Comp. Ex. 5** |
|---|---|---|---|---|
| Suds Mileage Index (Greasy Soil) | 123 | 129 | 133 | 117 |

All percentages and ratios given for enzymes are based on active protein. All percentages and ratios herein are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A detergent composition comprising a modified protein selected from the group consisting of modified ovalbumin, modified ovomucin, modified ovoglobulin, and mixtures thereof, and a surfactant system, preferably:

    a) said modified ovalbumin is derived from an unmodified ovalbumin which has at least at least 50%, preferably

at least 60%, preferably at least 70%, preferably at least 80%, preferably at least 85%, preferably at least 90%, preferably at least 95%, preferably at least 98% or even 100% amino acid identity to a native ovalbumin protein (SEQ ID NO: 1);

b) said modified ovomucin is derived from an unmodified ovomucin which has at least 50%, preferably at least 60%, preferably at least 70%, preferably at least 80%, preferably at least 85%, preferably at least 90%, preferably at least 95%, preferably at least 98% or even 100% amino acid identity to at least one native ovomucin protein selected from the group consisting of SEQ ID NO: 2, SEQ ID NO: 4, and SEQ ID NO: 5, preferably SEQ ID NO: 2 or SEQ ID NO: 5; and

c) said modified ovoglobulin is derived from an unmodified ovoglobulin which has at least 50%, preferably at least 60%, preferably at least 70%, preferably at least 80%, preferably at least 85%, preferably at least 90%, preferably at least 95%, preferably at least 98% or even 100% amino acid identity to a native ovoglobulin protein (SEQ ID NO: 3).

**2.** The composition according to claim 1, wherein said modified protein is selected from the group consisting of: (i) chemically modified protein(ii) physically modified protein and () combinations thereof.

**3.** The composition according to any preceding claims, wherein said modified protein is partially or completely denatured protein, preferably said denatured protein has been denatured by:

i) application of heat to said protein at a temperature of from 60°C to 100°C;
ii) pH treatment of said protein to a pH of from 1 to 4 or from 9 to 13;
iii) chemical denaturation of said protein;
iv) subjecting said protein to sonication, preferably high frequency sonication, in a range of from 5 kHz to 50 kHz; or
v) combinations of i) to iv).

**4.** The composition according to any preceding claims, wherein said modified protein is derived from partial or complete desugarization of said protein, preferably said desugarization being performed with enzyme, bacteria or yeast, preferably by *S. cerevisiae.*

**5.** The composition according to any preceding claims, wherein said modified protein is derived from partial or complete esterification of said protein with C1-C12 linear, cyclic or aromatic alcohol.

**6.** The composition according to any preceding claims, wherein said modified protein is a partially hydrolyzed protein, preferably derived from partial hydrolysis of said protein, preferably said partial hydrolysis being performed enzymatically by protease and said degree of hydrolyzation is 50% or less, preferably 20% or less, more preferably 10% or less, preferably said protease is papain.

**7.** The composition according to any preceding claims, wherein said modified protein is present in an amount of from 0.01 wt% to 5 wt%, preferably from 0.1% to 2.5 wt%, by weight of said composition.

**8.** The composition according to any preceding claims, wherein said modified protein comprises:

a) from 60 wt% to 95 wt%, preferably from 75% to 90 wt%, by weight of said modified protein of said modified ovalbumin;
b) from 1 wt% to 20 wt%, preferably from 2 wt% to 10 wt%, by weight of said modified protein of said modified ovomucin; and
c) from 1 wt% to 20 wt%, preferably from 5 wt% to 15 wt%, by weight of said modified protein of said modified ovoglobulin.

**9.** The composition according to any preceding claims, wherein said composition has a pH of from 7 to 10, preferably from 8.5 to 9, when measured at a 0.12% finished product composition in demineralized water at 20°C.

**10.** The composition according to any preceding claims, wherein said surfactant system is present in an amount of from 1 wt% to 60 wt%, preferably from 5 wt% to 40 wt%, by weight of said composition, and wherein said surfactant system comprises one or more anionic surfactants and one or more co-surfactants and wherein the weight ratio of said anionic surfactants to said co-surfactants is less than 9:1, more preferably from 5:1 to 1:1, more preferably from 4:1 to 2:1.

**11.** The composition according to claim 10, wherein said co-surfactants are selected from the group consisting of amphoteric surfactant, zwitterionic surfactant, and mixtures thereof, preferably said amphoteric surfactant is amine oxide surfactant and said zwitterionic surfactant is betaine surfactant.

**12.** The composition according to claims 10 or 11, wherein said anionic surfactants are a mixture of alkyl sulfates and alkyl alkoxy sulfates, wherein said co-surfactants are alkyl dimethyl amine oxides, and wherein the weight ratio of said anionic surfactants to said co-surfactants is from 4:1 to 2:1.

**13.** The composition according to any preceding claims further comprising a multivalent metal cation, preferably said multivalent metal cation is magnesium, aluminum, copper, calcium or iron, more preferably magnesium, most preferably said multivalent salt is magnesium chloride present in the amount of from 0.01 wt% to 2 wt% by weight of said composition.

**14.** A method of manually washing dishware comprising the steps of delivering a detergent composition according to any preceding claims into a volume of water to form a wash solution and immersing said dishware in said solution.

**15.** Use of a modified protein selected from the group consisting of modified ovalbumin, modified ovomucin, modified ovoglobulin, and mixtures thereof to provide increased suds longevity or increased grease emulsification in an aqueous wash liquor during a washing process, preferably:

a) said modified ovalbumin is derived from an unmodified ovalbumin which has at least at least 50%, preferably at least 60%, preferably at least 70%, preferably at least 80%, preferably at least 85%, preferably at least 90%, preferably at least 95%, preferably at least 98% or even 100% amino acid identity to a native ovalbumin protein (SEQ ID NO: 1);
b) said modified ovomucin is derived from an unmodified ovomucin which has at least 50%, preferably at least 60%, preferably at least 70%, preferably at least 80%, preferably at least 85%, preferably at least 90%, preferably at least 95%, preferably at least 98% or even 100% amino acid identity to at least one native ovomucin protein selected from the group consisting of SEQ ID NO: 2, SEQ ID NO: 4, and SEQ ID NO: 5, preferably SEQ ID NO: 2 or SEQ ID NO: 5; and
c) said modified ovoglobulin is derived from an unmodified ovoglobulin which has at least 50%, preferably at least 60%, preferably at least 70%, preferably at least 80%, preferably at least 85%, preferably at least 90%, preferably at least 95%, preferably at least 98% or even 100% amino acid identity to a native ovoglobulin protein (SEQ ID NO: 3).

Europäisches Patentamt
European Patent Office
Office européen des brevets

# EUROPEAN SEARCH REPORT

Application Number
EP 17 20 1323

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GB 1 551 074 A (PROCTER & GAMBLE) 22 August 1979 (1979-08-22) | 1-3,5, 7-10, 13-15 | INV. C11D1/94 C07K14/77 C07K14/46 C11D3/38 C11D11/00 |
| Y | * page 1, line 33 - page 2, line 4 * * page 3, lines 36-40 * * page 3, line 61 - page 5, line 4 * * page 6, lines 31-33, 53-55; claims 1,15; example IV; table IV * ----- | 4-6, 11-13 | |
| X | GB 1 529 841 A (PROCTER & GAMBLE LTD) 25 October 1978 (1978-10-25) | 1-3, 7-12,14, 15 | |
| Y | * page 1, line 40 - page 2, line 19; claims 1,6 * * page 4, lines 56-59 * * page 7, line 8 - page 8, line 45 * * page 9, lines 33-64; examples * ----- | 4-6, 11-13 | |
| X | K Rani ET AL: "Biodegradation of chemically modified egg albumin micropreparation for controlled release of bound Vigna mungo amylase and their application in fabric desizing as cost effective bio-active preparation", International Journal of Pharma and Bio Sciences, 1 January 2015 (2015-01-01), pages 1101-1111, XP055473525, Retrieved from the Internet: URL:https://pdfs.semanticscholar.org/42ab/fa15f7a052b1520c18539b7b258265bc1402.pdf?_ga=2.103685891.2034166414.1525781381-1900642341.1525781381 [retrieved on 2018-05-08] * page 1106 * ----- -/-- | 1-3,7, 9-13 | TECHNICAL FIELDS SEARCHED (IPC) C11D C07K |

The present search report has been drawn up for all claims

| Place of search | Date of completion of the search | Examiner |
|---|---|---|
| The Hague | 11 May 2018 | Marttin, Emmeline |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 17 20 1323

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JOLAN DE GROOT ET AL: "Deglycosylation of ovalbumin prohibits formation of a heat-stable conformer", BIOTECHNOLOGY AND BIOENGINEERING, vol. 97, no. 4, 8 December 2006 (2006-12-08), pages 735-741, XP055473631, US ISSN: 0006-3592, DOI: 10.1002/bit.21264 * abstract * ----- | 4 | |
| T | Anonymous: "Egg white - Wikipedia", Wikipedia, 30 December 2017 (2017-12-30), pages 1-5, XP055473613, Retrieved from the Internet: URL:https://en.wikipedia.org/wiki/Egg_white [retrieved on 2018-05-08] * page 2; table 1 * ----- | 8 | |
| Y | HAMMERSHOJ M ET AL: "Enzymatic hydrolysis of ovomucin and effect on foaming properties", FOOD RESEARCH INTERNATIONAL, ELSEVIER, AMSTERDAM, NL, vol. 41, no. 5, 1 January 2008 (2008-01-01), pages 522-531, XP022696090, ISSN: 0963-9969, DOI: 10.1016/J.FOODRES.2008.03.004 [retrieved on 2008-03-18] * abstract * ----- | 6 | TECHNICAL FIELDS SEARCHED (IPC) |

The present search report has been drawn up for all claims

2

| Place of search | Date of completion of the search | Examiner |
|---|---|---|
| The Hague | 11 May 2018 | Marttin, Emmeline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 20 1323

This annex lists the patent family members relating to the patent documents cited in the above-mentioned European search report. The members are as contained in the European Patent Office EDP file on
The European Patent Office is in no way liable for these particulars which are merely given for the purpose of information.

11-05-2018

| Patent document cited in search report | Publication date | Patent family member(s) | Publication date |
|---|---|---|---|
| GB 1551074 A | 22-08-1979 | DE 2636967 A1 | 03-03-1977 |
| | | FR 2321538 A1 | 18-03-1977 |
| | | GB 1551074 A | 22-08-1979 |
| | | IT 1064960 B | 25-02-1985 |
| | | JP S5252908 A | 28-04-1977 |
| | | NL 7609221 A | 22-02-1977 |
| GB 1529841 A | 25-10-1978 | CA 1059002 A | 24-07-1979 |
| | | GB 1529841 A | 25-10-1978 |
| | | JP S51125406 A | 01-11-1976 |
| | | PH 13399 A | 28-03-1980 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4434089 A
- GB 1160485 A
- WO 201219844 A
- WO 201219849 A
- WO 201219848 A
- US 3915903 A
- WO 2007135645 A


### Non-patent literature cited in the description


- **NEEDLEMAN ; WUNSCH.** *J. Mol. Biol.,* 1970, vol. 48, 443-453
- **HENIKOFF S. ; HENIKOFF J.G.** *P.N.A.S. USA,* 1992, vol. 89, 10915-10919
- **PETERSEN TN. ; BRUNAK S. ; VON HEIJNE G. ; NIELSEN H.** *Nature Methods,* 2011, vol. 8, 785-786
- **ROBERT LAUGHLIN.** The Aqueous Phase Behaviour of Surfactants. Academic Press, 1994, 538-542

SEQUENCE LISTING

<110>  The Procter & Gamble Company

<120>  Detergent composition

<130>  CM04899F

<160>  5

<170>  PatentIn version 3.5

<210>  1
<211>  386
<212>  PRT
<213>  Ovalbumin

<400>  1

```
Met Gly Ser Ile Gly Ala Ala Ser Met Glu Phe Cys Phe Asp Val Phe
1               5                   10                  15

Lys Glu Leu Lys Val His His Ala Asn Glu Asn Ile Phe Tyr Cys Pro
            20                  25                  30

Ile Ala Ile Met Ser Ala Leu Ala Met Val Tyr Leu Gly Ala Lys Asp
        35                  40                  45

Ser Thr Arg Thr Gln Ile Asn Lys Val Val Arg Phe Asp Lys Leu Pro
    50                  55                  60

Gly Phe Gly Asp Ser Ile Glu Ala Gln Cys Gly Thr Ser Val Asn Val
65                  70                  75                  80

His Ser Ser Leu Arg Asp Ile Leu Asn Gln Ile Thr Lys Pro Asn Asp
                85                  90                  95

Val Tyr Ser Phe Ser Leu Ala Ser Arg Leu Tyr Ala Glu Glu Arg Tyr
            100                 105                 110

Pro Ile Leu Pro Glu Tyr Leu Gln Cys Val Lys Glu Leu Tyr Arg Gly
        115                 120                 125

Gly Leu Glu Pro Ile Asn Phe Gln Thr Ala Ala Asp Gln Ala Arg Glu
    130                 135                 140

Leu Ile Asn Ser Trp Val Glu Ser Gln Thr Asn Gly Ile Ile Arg Asn
145                 150                 155                 160

Val Leu Gln Pro Ser Ser Val Asp Ser Gln Thr Ala Met Val Leu Val
                165                 170                 175
```

```
Asn Ala Ile Val Phe Lys Gly Leu Trp Glu Lys Ala Phe Lys Asp Glu
            180                 185                 190

Asp Thr Gln Ala Met Pro Phe Arg Val Thr Glu Gln Glu Ser Lys Pro
        195                 200                 205

Val Gln Met Met Tyr Gln Ile Gly Leu Phe Arg Val Ala Ser Met Ala
    210                 215                 220

Ser Glu Lys Met Lys Ile Leu Glu Leu Pro Phe Ala Ser Gly Thr Met
225                 230                 235                 240

Ser Met Leu Val Leu Leu Pro Asp Glu Val Ser Gly Leu Glu Gln Leu
                245                 250                 255

Glu Ser Ile Ile Asn Phe Glu Lys Leu Thr Glu Trp Thr Ser Ser Asn
            260                 265                 270

Val Met Glu Glu Arg Lys Ile Lys Val Tyr Leu Pro Arg Met Lys Met
        275                 280                 285

Glu Glu Lys Tyr Asn Leu Thr Ser Val Leu Met Ala Met Gly Ile Thr
    290                 295                 300

Asp Val Phe Ser Ser Ser Ala Asn Leu Ser Gly Ile Ser Ser Ala Glu
305                 310                 315                 320

Ser Leu Lys Ile Ser Gln Ala Val His Ala Ala His Ala Glu Ile Asn
                325                 330                 335

Glu Ala Gly Arg Glu Val Val Gly Ser Ala Glu Ala Gly Val Asp Ala
            340                 345                 350

Ala Ser Val Ser Glu Glu Phe Arg Ala Asp His Pro Phe Leu Phe Cys
        355                 360                 365

Ile Lys His Ile Ala Thr Asn Ala Val Leu Phe Phe Gly Arg Cys Val
    370                 375                 380

Ser Pro
385

<210>  2
<211>  2040
<212>  PRT
<213>  ovomucin

<400>  2
```

```
Met Glu Ile Lys Lys Glu Arg Ser Phe Trp Ile Phe Cys Leu Ile Trp
1               5                   10                  15

Ser Phe Cys Lys Gly Lys Glu Pro Val Gln Ile Val Gln Val Ser Thr
            20                  25                  30

Val Gly Arg Ser Glu Cys Thr Thr Trp Gly Asn Phe His Phe His Thr
        35                  40                  45

Phe Asp His Val Lys Phe Thr Phe Pro Gly Thr Cys Thr Tyr Val Phe
    50                  55                  60

Ala Ser His Cys Asn Asp Ser Tyr Gln Asp Phe Asn Ile Lys Ile Arg
65                  70                  75                  80

Arg Ser Asp Lys Asn Ser His Leu Ile Tyr Phe Thr Val Thr Thr Asp
                85                  90                  95

Gly Val Ile Leu Glu Val Lys Glu Thr Gly Ile Thr Val Asn Gly Asn
            100                 105                 110

Gln Ile Pro Leu Pro Phe Ser Leu Lys Ser Ile Leu Ile Glu Asp Thr
        115                 120                 125

Cys Ala Tyr Phe Gln Val Thr Ser Lys Leu Gly Leu Thr Leu Lys Trp
    130                 135                 140

Asn Trp Ala Asp Thr Leu Leu Leu Asp Leu Glu Glu Thr Tyr Lys Glu
145                 150                 155                 160

Lys Ile Cys Gly Leu Cys Gly Asn Tyr Asp Gly Asn Lys Lys Asn Asp
                165                 170                 175

Leu Ile Leu Asp Gly Tyr Lys Met His Pro Arg Gln Phe Gly Asn Phe
            180                 185                 190

His Lys Val Glu Asp Pro Ser Glu Lys Cys Pro Asp Val Arg Pro Asp
        195                 200                 205

Asp His Thr Gly Arg His Pro Thr Glu Asp Asp Asn Arg Cys Ser Lys
    210                 215                 220

Tyr Lys Lys Met Cys Lys Lys Leu Leu Ser Arg Phe Gly Asn Cys Pro
225                 230                 235                 240

Lys Val Val Ala Phe Asp Asp Tyr Val Ala Thr Cys Thr Glu Asp Met
                245                 250                 255
```

```
Cys Asn Cys Val Val Asn Ser Ser Gln Ser Asp Leu Val Ser Ser Cys
            260                 265                 270

Ile Cys Ser Thr Leu Asn Gln Tyr Ser Arg Asp Cys Val Leu Ser Lys
        275                 280                 285

Gly Asp Pro Gly Glu Trp Arg Thr Lys Glu Leu Cys Tyr Gln Glu Cys
    290                 295                 300

Pro Ser Asn Met Glu Tyr Met Glu Cys Gly Asn Ser Cys Ala Asp Thr
305                 310                 315                 320

Cys Ala Asp Pro Glu Arg Ser Lys Ile Cys Lys Ala Pro Cys Thr Asp
                325                 330                 335

Gly Cys Phe Cys Pro Pro Gly Thr Ile Leu Asp Asp Leu Gly Gly Lys
            340                 345                 350

Lys Cys Val Pro Arg Asp Ser Cys Pro Cys Met Phe Gln Gly Lys Val
        355                 360                 365

Tyr Ser Ser Gly Gly Thr Tyr Ser Thr Pro Cys Gln Asn Cys Thr Cys
    370                 375                 380

Lys Gly Gly His Trp Ser Cys Ile Ser Leu Pro Cys Ser Gly Ser Cys
385                 390                 395                 400

Ser Ile Asp Gly Gly Phe His Ile Lys Thr Phe Asp Asn Lys Lys Phe
                405                 410                 415

Asn Phe His Gly Asn Cys His Tyr Val Leu Ala Lys Asn Thr Asp Asp
            420                 425                 430

Thr Phe Val Val Ile Gly Glu Ile Ile Gln Cys Gly Thr Ser Lys Thr
        435                 440                 445

Met Thr Cys Leu Lys Asn Val Leu Val Thr Leu Gly Arg Thr Thr Ile
    450                 455                 460

Lys Ile Cys Ser Cys Gly Ser Ile Tyr Met Asn Asn Phe Ile Val Lys
465                 470                 475                 480

Leu Pro Val Ser Lys Asp Gly Ile Thr Ile Phe Arg Pro Ser Thr Phe
                485                 490                 495

Phe Ile Lys Ile Leu Ser Ser Ala Gly Val Gln Ile Arg Val Gln Met
            500                 505                 510
```

```
Lys Pro Val Met Gln Leu Ser Ile Thr Val Asp His Ser Tyr Gln Asn
        515                 520                 525

Arg Thr Ser Gly Leu Cys Gly Asn Phe Asn Asn Ile Gln Thr Asp Asp
    530                 535                 540

Phe Arg Thr Ala Thr Gly Ala Val Glu Asp Ser Ala Ala Ala Phe Gly
545                 550                 555                 560

Asn Ser Trp Lys Thr Arg Ala Ser Cys Phe Asp Val Glu Asp Ser Phe
                565                 570                 575

Glu Asp Pro Cys Ser Asn Ser Val Asp Lys Glu Lys Phe Ala Gln His
            580                 585                 590

Trp Cys Ala Leu Leu Ser Asn Thr Ser Ser Thr Phe Ala Ala Cys His
        595                 600                 605

Ser Val Val Asp Pro Ser Val Tyr Ile Lys Arg Cys Met Tyr Asp Thr
    610                 615                 620

Cys Asn Ala Glu Lys Ser Glu Val Ala Leu Cys Ser Val Leu Ser Thr
625                 630                 635                 640

Tyr Ser Arg Asp Cys Ala Ala Ala Gly Met Thr Leu Lys Gly Trp Arg
                645                 650                 655

Gln Gly Ile Cys Asp Pro Ser Glu Glu Cys Pro Glu Thr Met Val Tyr
            660                 665                 670

Asn Tyr Ser Val Lys Tyr Cys Asn Gln Ser Cys Arg Ser Leu Asp Glu
        675                 680                 685

Pro Asp Pro Leu Cys Lys Val Gln Ile Ala Pro Met Glu Gly Cys Gly
    690                 695                 700

Cys Pro Glu Gly Thr Tyr Leu Asn Asp Glu Glu Glu Cys Val Thr Pro
705                 710                 715                 720

Asp Asp Cys Pro Cys Tyr Tyr Lys Gly Lys Ile Val Gln Pro Gly Asn
                725                 730                 735

Ser Phe Gln Glu Asp Lys Leu Leu Cys Lys Cys Ile Gln Gly Arg Leu
            740                 745                 750

Asp Cys Ile Gly Glu Thr Val Leu Val Lys Asp Cys Pro Ala Pro Met
```

```
        755                 760                 765

Tyr Tyr Phe Asn Cys Ser Ser Ala Gly Pro Gly Ala Ile Gly Ser Glu
    770                 775                 780

Cys Gln Lys Ser Cys Lys Thr Gln Asp Met His Cys Tyr Val Thr Glu
785                 790                 795                 800

Cys Val Ser Gly Cys Met Cys Pro Asp Gly Leu Val Leu Asp Gly Ser
                805                 810                 815

Gly Gly Cys Ile Pro Lys Asp Gln Cys Pro Cys Val His Gly Gly His
            820                 825                 830

Phe Tyr Lys Pro Gly Glu Thr Ile Arg Val Asp Cys Asn Thr Cys Thr
        835                 840                 845

Cys Asn Lys Arg Gln Trp Asn Cys Thr Asp Asn Pro Cys Lys Gly Thr
    850                 855                 860

Cys Thr Val Tyr Gly Asn Gly His Tyr Met Ser Phe Asp Gly Glu Lys
865                 870                 875                 880

Phe Asp Phe Leu Gly Asp Cys Asp Tyr Ile Leu Ala Gln Asp Phe Cys
                885                 890                 895

Pro Asn Asn Met Asp Ala Gly Thr Phe Arg Ile Val Ile Gln Asn Asn
            900                 905                 910

Ala Cys Gly Lys Ser Leu Ser Ile Cys Ser Leu Lys Ile Thr Leu Ile
        915                 920                 925

Phe Glu Ser Ser Glu Ile Arg Leu Leu Glu Gly Arg Ile Gln Glu Ile
    930                 935                 940

Ala Thr Asp Pro Gly Ala Glu Lys Asn Tyr Lys Val Asp Leu Arg Gly
945                 950                 955                 960

Gly Tyr Ile Val Ile Glu Thr Thr Gln Gly Met Ser Phe Met Trp Asp
                965                 970                 975

Gln Lys Thr Thr Val Val Val His Val Thr Pro Ser Phe Gln Gly Lys
            980                 985                 990

Val Cys Gly Leu Cys Gly Asp Phe  Asp Gly Arg Ser Arg  Asn Asp Phe
        995                 1000                1005
```

```
Thr Thr  Arg Gly Gln Ser Val  Glu Met Ser Ile Gln  Glu Phe Gly
    1010                 1015                 1020

Asn Ser  Trp Lys Ile Thr Ser  Thr Cys Ser Asn Ile  Asn Met Thr
    1025                 1030                 1035

Asp Leu  Cys Ala Asp Gln Pro  Phe Lys Ser Ala Leu  Gly Gln Lys
    1040                 1045                 1050

His Cys  Ser Ile Ile Lys Ser  Ser Val Phe Glu Ala  Cys His Ser
    1055                 1060                 1065

Lys Val  Asn Pro Ile Pro Tyr  Tyr Glu Ser Cys Val  Ser Asp Phe
    1070                 1075                 1080

Cys Gly  Cys Asp Ser Val Gly  Asp Cys Glu Cys Phe  Cys Thr Ser
    1085                 1090                 1095

Val Ala  Ala Tyr Ala Arg Ser  Cys Ser Thr Ala Gly  Val Cys Ile
    1100                 1105                 1110

Asn Trp  Arg Thr Pro Ala Ile  Cys Pro Val Phe Cys  Asp Tyr Tyr
    1115                 1120                 1125

Asn Pro  Pro Asp Lys His Glu  Trp Phe Tyr Lys Pro  Cys Gly Ala
    1130                 1135                 1140

Pro Cys  Leu Lys Thr Cys Arg  Asn Pro Gln Gly Lys  Cys Gly Asn
    1145                 1150                 1155

Ile Leu  Tyr Ser Leu Glu Gly  Cys Tyr Pro Glu Cys  Ser Pro Asp
    1160                 1165                 1170

Lys Pro  Tyr Phe Asp Glu Glu  Arg Arg Glu Cys Val  Ser Leu Pro
    1175                 1180                 1185

Asp Cys  Thr Ser Cys Asn Pro  Glu Glu Lys Leu Cys  Thr Glu Asp
    1190                 1195                 1200

Ser Lys  Asp Cys Leu Cys Cys  Tyr Asn Gly Lys Thr  Tyr Pro Leu
    1205                 1210                 1215

Asn Glu  Thr Ile Tyr Ser Gln  Thr Glu Gly Thr Lys  Cys Gly Asn
    1220                 1225                 1230

Ala Phe  Cys Gly Pro Asn Gly  Met Ile Ile Glu Thr  Phe Ile Pro
    1235                 1240                 1245
```

Cys Ser Thr Leu Ser Val Pro Ala Gln Glu Gln Leu Met Gln Pro
1250 1255 1260

Val Thr Ser Ala Pro Leu Leu Ser Thr Glu Ala Thr Pro Cys Phe
1265 1270 1275

Cys Thr Asp Asn Gly Gln Leu Ile Gln Met Gly Glu Asn Val Ser
1280 1285 1290

Leu Pro Met Asn Ile Ser Gly His Cys Ala Tyr Ser Ile Cys Asn
1295 1300 1305

Ala Ser Cys Gln Ile Glu Leu Ile Trp Ala Glu Cys Lys Val Val
1310 1315 1320

Gln Thr Glu Ala Leu Glu Thr Cys Glu Pro Asn Ser Glu Ala Cys
1325 1330 1335

Pro Pro Thr Ala Ala Pro Asn Ala Thr Ser Leu Val Pro Ala Thr
1340 1345 1350

Ala Leu Ala Pro Met Ser Asp Cys Leu Gly Leu Ile Pro Pro Arg
1355 1360 1365

Lys Phe Asn Glu Ser Trp Asp Phe Gly Asn Cys Gln Ile Ala Thr
1370 1375 1380

Cys Leu Gly Glu Glu Asn Asn Ile Lys Leu Ser Ser Ile Thr Cys
1385 1390 1395

Pro Pro Gln Gln Leu Lys Leu Cys Val Asn Gly Phe Pro Phe Met
1400 1405 1410

Lys His His Asp Glu Thr Gly Cys Cys Glu Val Phe Glu Cys Gln
1415 1420 1425

Cys Ile Cys Ser Gly Trp Gly Asn Glu His Tyr Val Thr Phe Asp
1430 1435 1440

Gly Thr Tyr Tyr His Phe Lys Glu Asn Cys Thr Tyr Val Leu Val
1445 1450 1455

Glu Leu Ile Gln Pro Ser Ser Glu Lys Phe Trp Ile His Ile Asp
1460 1465 1470

Asn Tyr Tyr Cys Gly Ala Ala Asp Gly Ala Ile Cys Ser Met Ser
1475 1480 1485

Leu Leu Ile Phe His Ser Asn Ser Leu Val Ile Leu Thr Gln Ala
1490 1495 1500

Lys Glu His Gly Lys Gly Thr Asn Leu Val Leu Phe Asn Asp Lys
1505 1510 1515

Lys Val Val Pro Asp Ile Ser Lys Asn Gly Ile Arg Ile Thr Ser
1520 1525 1530

Ser Gly Leu Tyr Ile Ile Val Glu Ile Pro Glu Leu Glu Val Tyr
1535 1540 1545

Val Ser Tyr Ser Arg Leu Ala Phe Tyr Ile Lys Leu Pro Phe Gly
1550 1555 1560

Lys Tyr Tyr Asn Asn Thr Met Gly Leu Cys Gly Thr Cys Thr Asn
1565 1570 1575

Gln Lys Ser Asp Asp Ala Arg Lys Arg Asn Gly Glu Val Thr Asp
1580 1585 1590

Ser Phe Lys Glu Met Ala Leu Asp Trp Lys Ala Pro Val Ser Thr
1595 1600 1605

Asn Arg Tyr Cys Asn Pro Gly Ile Ser Glu Pro Val Lys Ile Glu
1610 1615 1620

Asn Tyr Gln His Cys Glu Pro Ser Glu Leu Cys Lys Ile Ile Trp
1625 1630 1635

Asn Leu Thr Glu Cys His Arg Val Val Pro Pro Gln Pro Tyr Tyr
1640 1645 1650

Glu Ala Cys Val Ala Ser Arg Cys Ser Gln Gln His Pro Ser Thr
1655 1660 1665

Glu Cys Gln Ser Met Gln Thr Tyr Ala Ala Leu Cys Gly Leu His
1670 1675 1680

Gly Ile Cys Val Asp Trp Arg Gly Gln Thr Asn Gly Gln Cys Glu
1685 1690 1695

Ala Thr Cys Ala Arg Asp Gln Val Tyr Lys Pro Cys Gly Glu Ala
1700 1705 1710

Lys Arg Asn Thr Cys Phe Ser Arg Glu Val Ile Val Asp Thr Leu

1715 1720 1725

Leu Ser Arg Asn Asn Thr Pro Val Phe Val Glu Gly Cys Tyr Cys
1730 1735 1740

Pro Asp Gly Asn Ile Leu Leu Asn Glu His Asp Gly Ile Cys Val
1745 1750 1755

Ser Val Cys Gly Cys Thr Ala Gln Asp Gly Ser Val Lys Lys Pro
1760 1765 1770

Arg Glu Ala Trp Glu His Asp Cys Gln Tyr Cys Thr Cys Asp Glu
1775 1780 1785

Glu Thr Leu Asn Ile Ser Cys Phe Pro Arg Pro Cys Ala Lys Ser
1790 1795 1800

Pro Pro Ile Asn Cys Thr Lys Glu Gly Phe Val Arg Lys Ile Lys
1805 1810 1815

Pro Arg Leu Asp Asp Pro Cys Cys Thr Glu Thr Val Cys Glu Cys
1820 1825 1830

Asp Ile Lys Thr Cys Ile Ile Asn Lys Thr Ala Cys Asp Leu Gly
1835 1840 1845

Phe Gln Pro Val Val Ala Ile Ser Glu Asp Gly Cys Cys Pro Ile
1850 1855 1860

Phe Ser Cys Ile Pro Lys Gly Val Cys Val Ser Glu Gly Val Glu
1865 1870 1875

Phe Lys Pro Gly Ala Val Val Pro Lys Ser Ser Cys Glu Asp Cys
1880 1885 1890

Val Cys Thr Asp Glu Gln Asp Ala Val Thr Gly Thr Asn Arg Ile
1895 1900 1905

Gln Cys Val Pro Val Lys Cys Gln Thr Thr Cys Gln Gln Gly Phe
1910 1915 1920

Arg Tyr Val Glu Lys Glu Gly Gln Cys Cys Ser Gln Cys Gln Gln
1925 1930 1935

Val Ala Cys Val Ala Asn Phe Pro Phe Gly Ser Val Thr Ile Glu
1940 1945 1950

Val Gly Lys Ser Tyr Lys Ala Pro Tyr Asp Asn Cys Thr Gln Tyr
1955 1960 1965

Thr Cys Thr Glu Ser Gly Gly Gln Phe Ser Leu Thr Ser Thr Val
1970 1975 1980

Lys Val Cys Leu Pro Phe Glu Glu Ser Asn Cys Val Pro Gly Thr
1985 1990 1995

Val Asp Val Thr Ser Asp Gly Cys Cys Lys Thr Cys Ile Asp Leu
2000 2005 2010

Pro His Lys Cys Lys Arg Ser Met Lys Glu Gln Tyr Ile Val His
2015 2020 2025

Lys His Cys Lys Ser Ala Ala Pro Val Pro Val Pro
2030 2035 2040

<210> 3
<211> 439
<212> PRT
<213> ovoglobulin

<400> 3

Met Gly Ala Leu Leu Ala Leu Leu Asp Pro Val Gln Pro Thr Arg Ala
1 5 10 15

Pro Asp Cys Gly Gly Ile Leu Thr Pro Leu Gly Leu Ser Tyr Leu Ala
20 25 30

Glu Val Ser Lys Pro His Ala Glu Val Val Leu Arg Gln Asp Leu Met
35 40 45

Ala Gln Arg Ala Ser Asp Leu Phe Leu Gly Ser Met Glu Pro Ser Arg
50 55 60

Asn Arg Ile Thr Ser Val Lys Val Ala Asp Leu Trp Leu Ser Val Ile
65 70 75 80

Pro Glu Ala Gly Leu Arg Leu Gly Ile Glu Val Glu Leu Arg Ile Ala
85 90 95

Pro Leu His Ala Val Pro Met Pro Val Arg Ile Ser Ile Gln Ala Asp
100 105 110

Leu His Val Asp Met Gly Pro Asp Gly Asn Leu Gln Leu Leu Thr Ser
115 120 125

```
Ala Cys Arg Pro Thr Val Gln Ala Gln Ser Thr Arg Glu Ala Glu Ser
    130                 135                 140

Lys Ser Ser Arg Ser Ile Leu Asp Lys Val Val Asp Val Asp Lys Leu
145                 150                 155                 160

Cys Leu Asp Val Ser Lys Leu Leu Leu Phe Pro Asn Glu Gln Leu Met
                165                 170                 175

Ser Leu Thr Ala Leu Phe Pro Val Thr Pro Asn Cys Gln Leu Gln Tyr
            180                 185                 190

Leu Pro Leu Ala Ala Pro Val Phe Ser Lys Gln Gly Ile Thr Leu Ser
        195                 200                 205

Leu Gln Thr Thr Phe Gln Val Ala Gly Ala Val Val Pro Val Pro Val
    210                 215                 220

Ser Pro Val Pro Phe Ser Met Pro Glu Leu Ala Ser Thr Ser Thr Ser
225                 230                 235                 240

His Leu Ile Leu Ala Leu Ser Glu His Phe Tyr Thr Ser Leu Tyr Phe
                245                 250                 255

Thr Leu Glu Arg Ala Gly Ala Phe Asn Met Thr Ile Pro Ser Met Leu
            260                 265                 270

Thr Thr Ala Thr Leu Ala Gln Lys Ile Thr Gln Val Gly Ser Leu Tyr
        275                 280                 285

His Glu Asp Leu Pro Ile Thr Leu Ser Ala Ala Leu Arg Ser Ser Pro
    290                 295                 300

Arg Val Val Leu Glu Glu Gly Arg Ala Ala Leu Lys Leu Phe Leu Thr
305                 310                 315                 320

Val His Ile Gly Ala Gly Ser Pro Asp Phe Gln Ser Phe Leu Ser Val
                325                 330                 335

Ser Ala Asp Val Thr Ala Gly Leu Gln Leu Ser Val Ser Asp Thr Arg
            340                 345                 350

Met Met Ile Ser Thr Ala Val Ile Glu Asp Ala Glu Leu Ser Leu Ala
        355                 360                 365

Ala Ser Asn Val Gly Leu Val Arg Ala Ala Leu Leu Glu Glu Leu Phe
    370                 375                 380
```

```
Leu Ala Pro Val Cys Gln Gln Val Pro Ala Trp Met Asp Asp Val Leu
385                 390                 395                 400

Arg Glu Gly Val His Leu Pro His Leu Ser His Phe Thr Tyr Thr Asp
                405                 410                 415

Val Asn Val Val Val His Lys Asp Tyr Val Leu Val Pro Cys Lys Leu
            420                 425                 430

Lys Leu Arg Ser Thr Met Ala
        435


<210>  4
<211>  1185
<212>  PRT
<213>  beta-ovomucin (MUC6)

<400>  4

Cys Ser Thr Trp Gly Gly Gly His Phe Ser Thr Phe Asp Lys Tyr Gln
1               5                   10                  15

Tyr Asp Phe Thr Gly Thr Cys Asn Tyr Ile Phe Ala Thr Val Cys Asp
            20                  25                  30

Glu Ser Ser Pro Asp Phe Asn Ile Gln Phe Arg Arg Gly Leu Asp Lys
        35                  40                  45

Lys Ile Ala Arg Ile Ile Ile Glu Leu Gly Pro Ser Val Ile Ile Val
    50                  55                  60

Glu Lys Asp Ser Ile Ser Val Arg Ser Val Gly Val Ile Lys Leu Pro
65                  70                  75                  80

Tyr Ala Ser Asn Gly Ile Gln Ile Ala Pro Tyr Gly Arg Ser Val Arg
                85                  90                  95

Leu Val Ala Lys Leu Met Glu Met Glu Leu Val Val Met Trp Asn Asn
            100                 105                 110

Glu Asp Tyr Leu Met Val Leu Thr Glu Lys Lys Tyr Met Gly Lys Thr
        115                 120                 125

Cys Gly Met Cys Gly Asn Tyr Asp Gly Tyr Glu Leu Asn Asp Phe Val
    130                 135                 140

Ser Glu Gly Lys Leu Leu Asp Thr Tyr Lys Phe Ala Ala Leu Gln Lys
145                 150                 155                 160
```

```
Met Asp Asp Pro Ser Glu Ile Cys Leu Ser Glu Glu Ile Ser Ile Pro
                165                 170                 175

Ala Ile Pro His Lys Lys Tyr Ala Val Ile Cys Ser Gln Leu Leu Asn
            180                 185                 190

Leu Val Ser Pro Thr Cys Ser Val Pro Lys Asp Gly Phe Val Thr Arg
        195                 200                 205

Cys Gln Leu Asp Met Gln Asp Cys Ser Glu Pro Gly Gln Lys Asn Cys
    210                 215                 220

Thr Cys Ser Thr Leu Ser Glu Tyr Ser Arg Gln Cys Ala Met Ser His
225                 230                 235                 240

Gln Val Val Phe Asn Trp Arg Thr Glu Asn Phe Cys Ser Val Gly Lys
                245                 250                 255

Cys Ser Ala Asn Gln Ile Tyr Glu Glu Cys Gly Ser Pro Cys Ile Lys
            260                 265                 270

Thr Cys Ser Asn Pro Glu Tyr Ser Cys Ser Ser His Cys Thr Tyr Gly
        275                 280                 285

Cys Phe Cys Pro Glu Gly Thr Val Leu Asp Asp Ile Ser Lys Asn Arg
    290                 295                 300

Thr Cys Val His Leu Glu Gln Cys Pro Cys Thr Leu Asn Gly Glu Thr
305                 310                 315                 320

Tyr Ala Pro Gly Asp Thr Met Lys Ala Ala Cys Arg Thr Cys Lys Cys
                325                 330                 335

Thr Met Gly Gln Trp Asn Cys Lys Glu Leu Pro Cys Pro Gly Arg Cys
            340                 345                 350

Ser Leu Glu Gly Gly Ser Phe Val Thr Thr Phe Asp Ser Arg Ser Tyr
        355                 360                 365

Arg Phe His Gly Val Cys Thr Tyr Ile Leu Met Lys Ser Ser Ser Leu
    370                 375                 380

Pro His Asn Gly Thr Leu Met Ala Ile Tyr Glu Lys Ser Gly Tyr Ser
385                 390                 395                 400

His Ser Glu Thr Ser Leu Ser Ala Ile Ile Tyr Leu Ser Thr Lys Asp
                405                 410                 415
```

```
Lys Ile Val Ile Ser Gln Asn Glu Leu Leu Thr Asp Asp Asp Glu Leu
            420                 425                 430

Lys Arg Leu Pro Tyr Lys Ser Gly Asp Ile Thr Ile Phe Lys Gln Ser
        435                 440                 445

Ser Met Phe Ile Gln Met His Thr Glu Phe Gly Leu Glu Leu Val Val
    450                 455                 460

Gln Thr Ser Pro Val Phe Gln Ala Tyr Val Lys Val Ser Ala Gln Phe
465                 470                 475                 480

Gln Gly Arg Thr Leu Gly Leu Cys Gly Asn Tyr Asn Gly Asp Thr Thr
                485                 490                 495

Asp Asp Phe Met Thr Ser Met Asp Ile Thr Glu Gly Thr Ala Ser Leu
            500                 505                 510

Phe Val Asp Ser Trp Arg Ala Gly Asn Cys Leu Pro Ala Met Glu Arg
        515                 520                 525

Glu Thr Asp Pro Cys Ala Leu Ser Gln Leu Asn Lys Ile Ser Ala Glu
    530                 535                 540

Thr His Cys Ser Ile Leu Thr Lys Lys Gly Thr Val Phe Glu Thr Cys
545                 550                 555                 560

His Ala Val Val Asn Pro Thr Pro Phe Tyr Lys Arg Cys Val Tyr Gln
                565                 570                 575

Ala Cys Asn Tyr Glu Glu Thr Phe Pro Tyr Ile Cys Ser Ala Leu Gly
            580                 585                 590

Ser Tyr Ala Arg Thr Cys Ser Ser Met Gly Leu Ile Leu Glu Asn Trp
        595                 600                 605

Arg Asn Ser Met Asp Asn Cys Thr Ile Thr Cys Thr Gly Asn Gln Thr
    610                 615                 620

Phe Ser Tyr Asn Thr Gln Ala Cys Glu Arg Thr Cys Leu Ser Leu Ser
625                 630                 635                 640

Asn Pro Thr Leu Glu Cys His Pro Thr Asp Ile Pro Ile Glu Gly Cys
                645                 650                 655

Asn Cys Pro Lys Gly Met Tyr Leu Asn His Lys Asn Glu Cys Val Arg
```

660 665 670

Lys Ser His Cys Pro Cys Tyr Leu Glu Asp Arg Lys Tyr Ile Leu Pro
675 680 685

Asp Gln Ser Thr Met Thr Gly Gly Ile Thr Cys Tyr Cys Val Asn Gly
690 695 700

Arg Leu Ser Cys Thr Gly Lys Leu Gln Asn Pro Ala Glu Ser Cys Lys
705 710 715 720

Ala Pro Lys Lys Tyr Ile Ser Cys Ser Asp Ser Leu Glu Asn Lys Tyr
725 730 735

Gly Ala Thr Cys Ala Pro Thr Cys Gln Met Leu Ala Thr Gly Ile Glu
740 745 750

Cys Ile Pro Thr Lys Cys Glu Ser Gly Cys Val Cys Ala Asp Gly Leu
755 760 765

Tyr Glu Asn Leu Asp Gly Arg Cys Val Pro Pro Glu Glu Cys Pro Cys
770 775 780

Glu Tyr Gly Gly Leu Ser Tyr Gly Lys Gly Glu Gln Ile Gln Thr Glu
785 790 795 800

Cys Glu Ile Cys Thr Cys Arg Lys Gly Lys Trp Lys Cys Val Gln Lys
805 810 815

Ser Arg Cys Ser Ser Thr Cys Asn Leu Tyr Gly Glu Gly His Ile Thr
820 825 830

Thr Phe Asp Gly Gln Arg Phe Val Phe Asp Gly Asn Cys Glu Tyr Ile
835 840 845

Leu Ala Met Asp Gly Cys Asn Val Asn Arg Pro Leu Ser Ser Phe Lys
850 855 860

Ile Val Thr Glu Asn Val Ile Cys Gly Lys Ser Gly Val Thr Cys Ser
865 870 875 880

Arg Ser Ile Ser Ile Tyr Leu Gly Asn Leu Thr Ile Ile Leu Arg Asp
885 890 895

Glu Thr Tyr Ser Ile Ser Gly Lys Asn Leu Gln Val Lys Tyr Asn Val
900 905 910

```
Lys Lys Asn Ala Leu His Leu Met Phe Asp Ile Ile Ile Pro Gly Lys
        915                 920                 925

Tyr Asn Met Thr Leu Ile Trp Asn Lys His Met Asn Phe Phe Ile Lys
    930                 935                 940

Ile Ser Arg Glu Thr Gln Glu Thr Ile Cys Gly Leu Cys Gly Asn Tyr
945                 950                 955                 960

Asn Gly Asn Met Lys Asp Asp Phe Glu Thr Arg Ser Lys Tyr Val Ala
                965                 970                 975

Ser Asn Glu Leu Glu Phe Val Asn Ser Trp Lys Glu Asn Pro Leu Cys
            980                 985                 990

Gly Asp Val Tyr Phe Val Val Asp  Pro Cys Ser Lys Asn  Pro Tyr Arg
        995                 1000                1005

Lys Ala  Trp Ala Glu Lys Thr  Cys Ser Ile Ile Asn  Ser Gln Val
    1010                1015                1020

Phe Ser  Ala Cys His Asn Lys  Val Asn Arg Met Pro  Tyr Tyr Glu
    1025                1030                1035

Ala Cys  Val Arg Asp Ser Cys  Gly Cys Asp Ile Gly  Gly Asp Cys
    1040                1045                1050

Glu Cys  Met Cys Asp Ala Ile  Ala Val Tyr Ala Met  Ala Cys Leu
    1055                1060                1065

Asp Lys  Gly Ile Cys Ile Asp  Trp Arg Thr Pro Glu  Phe Cys Pro
    1070                1075                1080

Val Tyr  Cys Glu Tyr Tyr Asn  Ser His Arg Lys Thr  Gly Ser Gly
    1085                1090                1095

Gly Ala  Tyr Ser Tyr Gly Ser  Ser Val Asn Cys Thr  Trp His Tyr
    1100                1105                1110

Arg Pro  Cys Asn Cys Pro Asn  Gln Tyr Tyr Lys Tyr  Val Asn Ile
    1115                1120                1125

Glu Gly  Cys Tyr Asn Cys Ser  His Asp Glu Tyr Phe  Asp Tyr Glu
    1130                1135                1140

Lys Glu  Lys Cys Met Pro Cys  Ala Met Gln Pro Thr  Ser Val Thr
    1145                1150                1155
```

```
Leu Pro  Thr Ala Thr Gln Pro  Thr Ser Pro Ser Thr  Ser Ser Ala
    1160                 1165                 1170

Ser Thr  Val Leu Thr Glu Thr  Thr Asn Pro Pro Val
    1175                 1180                 1185
```

<210> 5
<211> 900
<212> PRT
<213> alpha-ovomucin (MUC5B)

<400> 5

```
Met Glu Ile Lys Lys Glu Arg Ser Phe Trp Ile Phe Cys Leu Ile Trp
1               5                   10                  15

Ser Phe Cys Lys Gly Lys Glu Pro Val Gln Ile Val Gln Val Ser Thr
            20                  25                  30

Val Gly Arg Ser Glu Cys Thr Thr Trp Gly Asn Phe His Phe His Thr
        35                  40                  45

Phe Asp His Val Lys Phe Thr Phe Pro Gly Thr Cys Thr Tyr Val Phe
    50                  55                  60

Ala Ser His Cys Asn Asp Ser Tyr Gln Asp Phe Asn Ile Lys Ile Arg
65                  70                  75                  80

Arg Ser Asp Lys Asn Ser His Leu Ile Tyr Phe Thr Val Thr Thr Asp
                85                  90                  95

Gly Val Ile Leu Glu Val Lys Glu Thr Gly Ile Thr Val Asn Gly Asn
            100                 105                 110

Gln Ile Pro Leu Pro Phe Ser Leu Lys Ser Ile Leu Ile Glu Asp Thr
        115                 120                 125

Cys Ala Tyr Phe Gln Val Thr Ser Lys Leu Gly Leu Thr Leu Lys Trp
    130                 135                 140

Asn Trp Ala Asp Thr Leu Leu Leu Asp Leu Glu Glu Thr Tyr Lys Glu
145                 150                 155                 160

Lys Ile Cys Gly Leu Cys Gly Asn Tyr Asp Gly Asn Lys Lys Asn Asp
                165                 170                 175

Leu Ile Leu Asp Gly Tyr Lys Met His Pro Arg Gln Phe Gly Asn Phe
            180                 185                 190
```

His Lys Val Glu Asp Pro Ser Glu Lys Cys Pro Asp Val Arg Pro Asp
195 200 205

Asp His Thr Gly Arg His Pro Thr Glu Asp Asp Asn Arg Cys Ser Lys
210 215 220

Tyr Lys Lys Met Cys Lys Lys Leu Leu Ser Arg Phe Gly Asn Cys Pro
225 230 235 240

Lys Val Val Ala Phe Asp Asp Tyr Val Ala Thr Cys Thr Glu Asp Met
245 250 255

Cys Asn Cys Val Val Asn Ser Ser Gln Ser Asp Leu Val Ser Ser Cys
260 265 270

Ile Cys Ser Thr Leu Asn Gln Tyr Ser Arg Asp Cys Val Leu Ser Lys
275 280 285

Gly Asp Pro Gly Glu Trp Arg Thr Lys Glu Leu Cys Tyr Gln Glu Cys
290 295 300

Pro Ser Asn Met Glu Tyr Met Glu Cys Gly Asn Ser Cys Ala Asp Thr
305 310 315 320

Cys Ala Asp Pro Glu Arg Ser Lys Ile Cys Lys Ala Pro Cys Thr Asp
325 330 335

Gly Cys Phe Cys Pro Pro Gly Thr Ile Leu Asp Asp Leu Gly Gly Lys
340 345 350

Lys Cys Val Pro Arg Asp Ser Cys Pro Cys Met Phe Gln Gly Lys Val
355 360 365

Tyr Ser Ser Gly Gly Thr Tyr Ser Thr Pro Cys Gln Asn Cys Thr Cys
370 375 380

Lys Gly Gly His Trp Ser Cys Ile Ser Leu Pro Cys Ser Gly Ser Cys
385 390 395 400

Ser Ile Asp Gly Gly Phe His Ile Lys Thr Phe Asp Asn Lys Lys Phe
405 410 415

Asn Phe His Gly Asn Cys His Tyr Val Leu Ala Lys Asn Thr Asp Asp
420 425 430

Thr Phe Val Val Ile Gly Glu Ile Ile Gln Cys Gly Thr Ser Lys Thr
435 440 445

Met Thr Cys Leu Lys Asn Val Leu Val Thr Leu Gly Arg Thr Thr Ile
450 455 460

Lys Ile Cys Ser Cys Gly Ser Ile Tyr Met Asn Asn Phe Ile Val Lys
465 470 475 480

Leu Pro Val Ser Lys Asp Gly Ile Thr Ile Phe Arg Pro Ser Thr Phe
485 490 495

Phe Ile Lys Ile Leu Ser Ser Ala Gly Val Gln Ile Arg Val Gln Met
500 505 510

Lys Pro Val Met Gln Leu Ser Ile Thr Val Asp His Ser Tyr Gln Asn
515 520 525

Arg Thr Ser Gly Leu Cys Gly Asn Phe Asn Asn Ile Gln Thr Asp Asp
530 535 540

Phe Arg Thr Ala Thr Gly Ala Val Glu Asp Ser Ala Ala Ala Phe Gly
545 550 555 560

Asn Ser Trp Lys Thr Arg Ala Ser Cys Phe Asp Val Glu Asp Ser Phe
565 570 575

Glu Asp Pro Cys Ser Asn Ser Val Asp Lys Glu Lys Phe Ala Gln His
580 585 590

Trp Cys Ala Leu Leu Ser Asn Thr Ser Ser Thr Phe Ala Ala Cys His
595 600 605

Ser Val Val Asp Pro Ser Val Tyr Ile Lys Arg Cys Met Tyr Asp Thr
610 615 620

Cys Asn Ala Glu Lys Ser Glu Val Ala Leu Cys Ser Val Leu Ser Thr
625 630 635 640

Tyr Ser Arg Asp Cys Ala Ala Ala Gly Met Thr Leu Lys Gly Trp Arg
645 650 655

Gln Gly Ile Cys Asp Pro Ser Glu Glu Cys Pro Glu Thr Met Val Tyr
660 665 670

Asn Tyr Ser Val Lys Tyr Cys Asn Gln Ser Cys Arg Ser Leu Asp Glu
675 680 685

Pro Asp Pro Leu Cys Lys Val Gln Ile Ala Pro Met Glu Gly Cys Gly

```
    690                 695                 700

Cys Pro Glu Gly Thr Tyr Leu Asn Asp Glu Glu Glu Cys Val Thr Pro
705                 710                 715                 720

Asp Asp Cys Pro Cys Tyr Tyr Lys Gly Lys Ile Val Gln Pro Gly Asn
                725                 730                 735

Ser Phe Gln Glu Asp Lys Leu Leu Cys Lys Cys Ile Gln Gly Arg Leu
            740                 745                 750

Asp Cys Ile Gly Glu Thr Val Leu Val Lys Asp Cys Pro Ala Pro Met
        755                 760                 765

Tyr Tyr Phe Asn Cys Ser Ser Ala Gly Pro Gly Ala Ile Gly Ser Glu
    770                 775                 780

Cys Gln Lys Ser Cys Lys Thr Gln Asp Met His Cys Tyr Val Thr Glu
785                 790                 795                 800

Cys Val Ser Gly Cys Met Cys Pro Asp Gly Leu Val Leu Asp Gly Ser
                805                 810                 815

Gly Gly Cys Ile Pro Lys Asp Gln Cys Pro Cys Val His Gly Gly His
            820                 825                 830

Phe Tyr Lys Pro Gly Glu Thr Ile Arg Val Asp Cys Asn Thr Cys Thr
        835                 840                 845

Cys Asn Lys Arg Gln Trp Asn Cys Thr Asp Asn Pro Cys Lys Gly Thr
    850                 855                 860

Cys Thr Val Tyr Gly Asn Gly His Tyr Met Ser Phe Asp Gly Glu Lys
865                 870                 875                 880

Phe Asp Phe Leu Gly Asp Cys Asp Tyr Ile Leu Ala Gln Asp Phe Cys
                885                 890                 895

Pro Asn Asn Met
            900
```